# EUROPEAN PATENT SPECIFICATION

(11) **EP 3 494 117 B1**
(45) Date of publication and mention of the grant of the patent: **28.10.2026**
(21) Application number: 17834782.9
(22) Date of filing: 27.07.2017
(51) Int. Cl.: C09K 11/06, C07D 487/16, H05B 33/14, H10K 50/11, H10K 85/60

(54) **ORGANIC ELECTROLUMINESCENT COMPOUND AND ORGANIC ELECTROLUMINESCENT DEVICE COMPRISING THE SAME**
ORGANISCHE ELEKTROLUMINESZENTE VERBINDUNG UND ORGANISCHE ELEKTROLUMINESZENTE VORRICHTUNG DAMIT
COMPOSÉ ÉLECTROLUMINESCENT ORGANIQUE ET DISPOSITIF ÉLECTROLUMINESCENT ORGANIQUE LE COMPRENANT

(30) Priority: 27.07.2016 KR 20160095622; 26.07.2017 KR 20170094723
(43) Date of publication of application: 12.06.2019
(73) Proprietor: DuPont Specialty Materials Korea Ltd., Cheonan-si, Chungcheongnam-do 31093 (KR)
(72) Inventor: MOON, Doo-Hyeon, Hwaseong-si Gyeonggi-do 18449 (KR); JUN, Ji-Song, Hwaseong-si Gyeonggi-do 18449 (KR)
(74) Representative: Houghton, Mark Phillip
(86) International application number: PCT/KR2017/008083
(87) International publication number: WO 2018/021841

(56) References cited:
- WO-A1-2016/080791
- WO-A1-2017/043770
- WO-A1-2017/095156
- US-A1- 2016 163 998

## Description

### Technical Field

The present disclosure relates to an organic electroluminescent compound and an organic electroluminescent device comprising the same.

### Background Art

Among display devices, an electroluminescent device (EL device) is a self-light-emitting device which has advantages in that it provides a wider viewing angle, a greater contrast ratio, and a faster response time. The first organic EL device was developed by Eastman Kodak in 1987, by using small aromatic diamine molecules and aluminum complexes as materials for forming a light-emitting layer [Appl. Phys. Lett. 51, 913, 1987].

The most important factor determining luminous efficiency in an organic electroluminescent device is light-emitting materials. Until now, fluorescent materials have been widely used as a light-emitting material. However, in view of electroluminescent mechanisms, since phosphorescent light-emitting materials theoretically enhance luminous efficiency by four (4) times compared to fluorescent light-emitting materials, development of phosphorescent light-emitting materials are widely being researched. To date, iridium(III) complexes have been widely known as phosphorescent materials, including bis(2-(2'-benzothienyl)-pyridinato-N,C3')iridium(acetylacetonate) ((acac)Ir(btp)₂), tris(2-phenylpyridine)iridium (Ir(ppy)₃) and bis(4,6-difluorophenylpyridinato-N,C2)picolinate iridium (Firpic) as red, green, and blue materials, respectively.

At present, 4,4'-N,N'-dicarbazol-biphenyl (CBP) is the most widely known phosphorescent host material. Recently, Pioneer (Japan) et al. developed a high performance organic electroluminescent device using bathocuproine (BCP) and aluminum(III)bis(2-methyl-8-quinolinate)(4-phenylphenolate) (BAlq) etc., which were used as hole blocking layer materials, as host materials.

Although these materials provide good light-emitting characteristics, they have the following disadvantages: (1) Due to their low glass transition temperature and poor thermal stability, their degradation may occur during a high-temperature deposition process in a vacuum, and the lifespan of the device decreases. (2) The power efficiency of an organic electroluminescent device is given by [(π/voltage) × current efficiency], and the power efficiency is inversely proportional to the voltage. Although an organic electroluminescent device comprising phosphorescent host materials provides higher current efficiency (cd/A) than one comprising fluorescent materials, a significantly high driving voltage is necessary. Thus, there is no merit in terms of power efficiency (Im/W). (3) Further, when these materials are used in an organic electroluminescent device, the operational lifespan of an organic electroluminescent device is short and luminous efficiency is still required to be improved.

In order to enhance luminous efficiency, driving voltage and/or lifespan, various materials or concepts for an organic layer of an organic electroluminescent device have been proposed. However, they were not satisfactory to use practically.

Korean Patent Appln. Laying-Open No. KR 2015-0077220 discloses a compound of a fused structure comprising a carbazole and an azepine as a compound for an organic electroluminescent device. However, said reference does not specifically disclose a compound of a fused structure comprising an indolocarbazole and an azepine. WO2016/080791A1 discloses a plurality of host materials and an organic electroluminescent device comprising the same to possible to produce an organic electroluminescent device having improved driving lifespan and excellent current efficiency and power efficiency compared to the organic electroluminescent device using the conventional sole host material. US2016/163998A1 discloses a nitrogen-containing heterocyclic compound wherein a pyrrole ring, an aromatic ring and a 7-membered ring are fused one another, a material for organic electroluminescence device including the compound, and an organic electroluminescence device including the material. WO2017/043770A1 discloses an organic electroluminescent compound and an organic electroluminescent device comprising the same having low driving voltage and good current and power efficiencies. WO2017/095156A1 discloses an organic electroluminescent compound and an organic electroluminescent device having a remarkably improved lifespan.

### Disclosure of the Invention

### Problems to be Solved

The objective of the present disclosure is to provide i) an organic electroluminescent compound which can produce an organic electroluminescent device having low driving voltage, high luminous efficiency, and/or excellent lifespan characteristic, and ii) an organic electroluminescent device comprising the compound.

### Solution to Problems

As a result of intensive studies to solve the technical problem above, the present inventors found that the above objective can be achieved by an organic electroluminescent compound represented by the following formula 1: 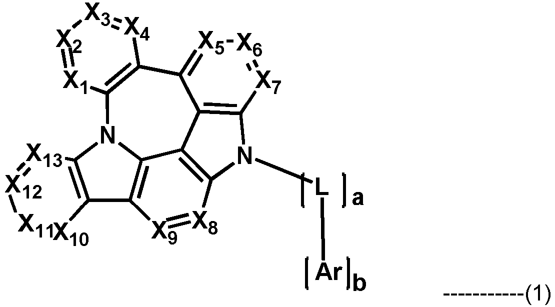 wherein
X₁ to X₁₀ each represent CR₁;
X₁₁ to X₁₃ each independently represent N or CR₁;
L represents a single bond, a substituted or unsubstituted (C6-C30)arylene, or a substituted or unsubstituted 3- to 30-membered heteroarylene;
Ar and R₁ each independently represent hydrogen, deuterium, a halogen, a cyano, a substituted or unsubstituted (C1-C30)alkyl, a substituted or unsubstituted (C6-C30)aryl, a substituted or unsubstituted 3- to 30-membered heteroaryl, a substituted or unsubstituted (C3-C30)cycloalkyl, a substituted or unsubstituted (C1-C30)alkoxy, a substituted or unsubstituted tri(C1-C30)alkylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted tri(C6-C30)arylsilyl, a substituted or unsubstituted mono- or di- (C1-C30)alkylamino, a substituted or unsubstituted mono- or di- (C6-C30)arylamino, or a substituted or unsubstituted (C1-C30)alkyl(C6-C30)arylamino; or are linked to an adjacent substituent to form a substituted or unsubstituted, mono- or polycyclic, (C3-C30) alicyclic or aromatic ring, whose carbon atom(s) may be replaced with at least one heteroatom selected from nitrogen, oxygen, and sulfur;
the heteroaryl(ene) contains at least one heteroatom selected from B, N, O, S, Si, and P; and
a and b each independently represent an integer of 1 to 2.

### Effects of the Invention

By using the organic electroluminescent compound according to the present disclosure, an organic electroluminescent device having low driving voltage, high luminous efficiency, and/or excellent lifespan characteristic can be produced.

### Brief Descriptions of the Figures

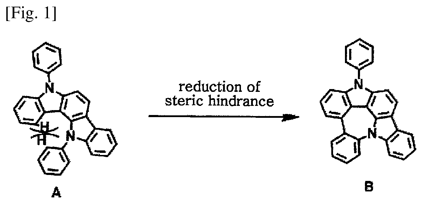

Figure 1 shows that the compound according to the present disclosure has a reduced steric hindrance compared to a conventional compound.

### Embodiments of the Invention

Hereinafter, the present disclosure will be described in detail. However, the following description is intended to explain the disclosure, and is not meant in any way to restrict the scope of the disclosure.

The present disclosure relates to an organic electroluminescent compound represented by formula 1, an organic electroluminescent material comprising the compound, and an organic electroluminescent device comprising the material.

The term "organic electroluminescent compound" in the present disclosure means a compound that may be used in an organic electroluminescent device, and may be comprised in any layer constituting an organic electroluminescent device, as necessary.

The term "organic electroluminescent material" in the present disclosure means a material that may be used in an organic electroluminescent device, and may comprise at least one compound. The organic electroluminescent material may be comprised in any layer constituting an organic electroluminescent device, as necessary. For example, the organic electroluminescent material may be a hole injection material, a hole transport material, a hole auxiliary material, a light-emitting auxiliary material, an electron blocking material, a light-emitting material, an electron buffer material, a hole blocking material, an electron transport material, or an electron injection material.

The organic electroluminescent material of the present disclosure may comprise at least one compound represented by formula 1. The compound represented by formula 1 may be comprised in at least one layer constituting an organic electroluminescent device, and may be comprised in a light-emitting layer, but is not limited thereto. When comprised in the light-emitting layer, it can be comprised as a phosphorescent host material.

Hereinafter, the organic electroluminescent compound represented by formula 1 will be described in detail.

The compound of formula 1 may be represented by any one of the following formulas 2 to 7: 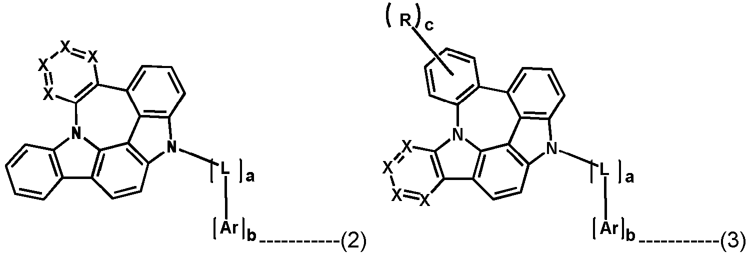 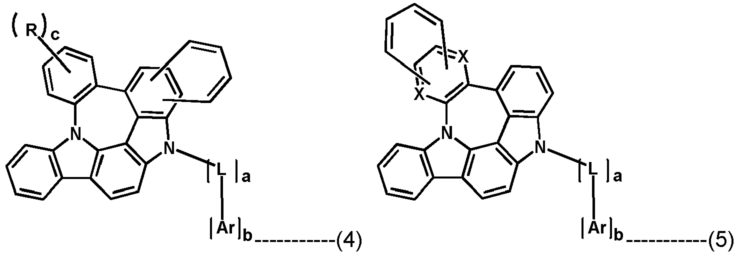 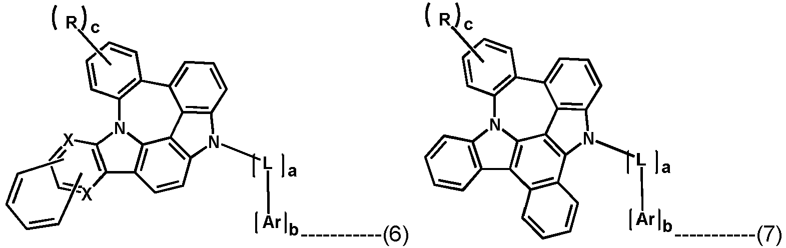 wherein
X represents CR₁;
R represents a substituted or unsubstituted mono- or di- (C6-C30)arylamino;
c represents an integer of 1 to 2; and
L, Ar, R₁, a, and b are as defined in formula 1.

Herein, "(C1-C30)alkyl(ene)" is meant to be a linear or branched alkyl(ene) having 1 to 30 carbon atoms, in which the number of carbon atoms is preferably 1 to 10, more preferably 1 to 6, and includes methyl, ethyl, n-propyl, isopropyl, n-butyl, isobutyl, tert-butyl, etc. "(C2-C30)alkenyl" is meant to be a linear or branched alkenyl having 2 to 30 carbon atoms, in which the number of carbon atoms is preferably 2 to 20, more preferably 2 to 10, and includes vinyl, 1-propenyl, 2-propenyl, 1-butenyl, 2-butenyl, 3-butenyl, 2-methylbut-2-enyl, etc. "(C2-C30)alkynyl" is meant to be a linear or branched alkynyl having 2 to 30 carbon atoms, in which the number of carbon atoms is preferably 2 to 20, more preferably 2 to 10, and includes ethynyl, 1-propynyl, 2-propynyl, 1-butynyl, 2-butynyl, 3-butynyl, 1-methylpent-2-ynyl, etc. "(C3-C30)cycloalkyl(ene)" is meant to be a mono- or polycyclic hydrocarbon having 3 to 30 carbon atoms, in which the number of carbon atoms is preferably 3 to 20, more preferably 3 to 7, and includes cyclopropyl, cyclobutyl, cyclopentyl, cyclohexyl, etc. "3-to 7-membered heterocycloalkyl" is meant to be a cycloalkyl having at least one heteroatom selected from the group consisting of B, N, O, S, Si, and P, preferably O, S, and N, and 3 to 7 ring backbone atoms, and includes tetrahydrofuran, pyrrolidine, thiolan, tetrahydropyran, etc. "(C6-C30)aryl(ene)" is meant to be a monocyclic or fused ring radical derived from an aromatic hydrocarbon having 6 to 30 ring backbone carbon atoms, in which the number of ring backbone carbon atoms is preferably 6 to 20, more preferably 6 to 15, and includes phenyl, biphenyl, terphenyl, naphthyl, binaphthyl, phenylnaphthyl, naphthylphenyl, phenylterphenyl, fluorenyl, phenylfluorenyl, benzofluorenyl, dibenzofluorenyl, phenanthrenyl, phenylphenanthrenyl, anthracenyl, indenyl, triphenylenyl, pyrenyl, tetracenyl, perylenyl, chrysenyl, naphthacenyl, fluoranthenyl, etc. "3- to 30-membered heteroaryl(ene)" is meant to be an aryl group having at least one, preferably 1 to 4 heteroatoms selected from the group consisting of B, N, O, S, Si, and P, and 3 to 30 ring backbone atoms; is a monocyclic ring, or a fused ring condensed with at least one benzene ring; may be partially saturated; may be one formed by linking at least one heteroaryl or aryl group to a heteroaryl group via a single bond(s); and includes a monocyclic ring-type heteroaryl including furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl, etc., and a fused ring-type heteroaryl including benzofuranyl, benzothiophenyl, isobenzofuranyl, dibenzofuranyl, dibenzothiophenyl, benzimidazolyl, benzothiazolyl, benzoisothiazolyl, benzoisoxazolyl, benzoxazolyl, isoindolyl, indolyl, indazolyl, benzothiadiazolyl, quinolyl, isoquinolyl, cinnolinyl, quinazolinyl, benzoquinazolinyl, quinoxalinyl, benzoquinoxalinyl, carbazolyl, benzocarbazolyl, phenoxazinyl, phenanthridinyl, benzodioxolyl, etc. "Halogen" includes F, Cl, Br, and I.

Herein, "substituted" in the expression "substituted or unsubstituted" means that a hydrogen atom in a certain functional group is replaced with another atom or functional group, i.e., a substituent. The substituents of the substituted (C1-C30)alkyl(ene), the substituted (C6-C30)aryl(ene), the substituted 3- to 30-membered heteroaryl(ene), the substituted (C3-C30)cycloalkyl(ene), the substituted (C1-C30)alkoxy, the substituted tri(C1-C30)alkylsilyl, the substituted di(C1-C30)alkyl(C6-C30)arylsilyl, the substituted (C1-C30)alkyldi(C6-C30)arylsilyl, the substituted tri(C6-C30)arylsilyl, the substituted mono- or di- (C1-C30)alkylamino, the substituted mono- or di- (C6-C30)arylamino, the substituted (C1-C30)alkyl(C6-C30)arylamino, and the substituted mono- or polycyclic, (C3-C30) alicyclic or aromatic ring in Ar, L, and R₁ each independently are at least one selected from the group consisting of deuterium, a halogen, a cyano, a carboxyl, a nitro, a hydroxyl, a (C1-C30)alkyl, a halo(C1-C30)alkyl, a (C2-C30)alkenyl, a (C2-C30)alkynyl, a (C1-C30)alkoxy, a (C1-C30)alkylthio, a (C3-C30)cycloalkyl, a (C3-C30)cycloalkenyl, a 3- to 7-membered heterocycloalkyl, a (C6-C30)aryloxy, a (C6-C30)arylthio, a 5- to 30-membered heteroaryl unsubstituted or substituted with a (C6-C30)aryl, a (C6-C30)aryl unsubstituted or substituted with a 5- to 30-membered heteroaryl, a tri(C1-C30)alkylsilyl, a tri(C6-C30)arylsilyl, a di(C1-C30)alkyl(C6-C30)arylsilyl, a (C1-C30)alkyldi(C6-C30)arylsilyl, an amino, a mono- or di-(C1-C30)alkylamino, a mono- or di- (C6-C30)arylamino unsubstituted or substituted with a (C1-C30)alkyl, a (C1-C30)alkyl(C6-C30)arylamino, a (C1-C30)alkylcarbonyl, a (C1-C30)alkoxycarbonyl, a (C6-C30)arylcarbonyl, a di(C6-C30)arylboronyl, a di(C1-C30)alkylboronyl, a (C1-C30)alkyl(C6-C30)arylboronyl, a (C6-C30)aryl(C1-C30)alkyl, and a (C1-C30)alkyl(C6-C30)aryl; and preferably each independently are at least one selected from the group consisting of a (C1-C6)alkyl; a (C6-C25)aryl unsubstituted or substituted with a (C1-C6)alkyl or a (C6-C12)aryl; a 5- to 20-membered heteroaryl unsubstituted or substituted with a (C6-C12)aryl; and a (C1-C6)alkyl (C6-C12)aryl.

In formula 1 above, X₁ to X₁₀ each represent CR₁ and X₁₁ to X₁₃ each independently represent N or CR₁.

L represents a single bond, a substituted or unsubstituted (C6-C30)arylene, or a substituted or unsubstituted 3- to 30-membered heteroarylene, or a substituted or unsubstituted (C3-C30)cycloalkylene. Preferably L represents a single bond, a substituted or unsubstituted (C6-C15)arylene, or a substituted or unsubstituted 5- to 15-membered heteroarylene, and more preferably represents a single bond, an unsubstituted (C6-C15)arylene, or an unsubstituted 5- to 15-membered heteroarylene.

Ar and R₁ each independently represent hydrogen, deuterium, a halogen, a cyano, a substituted or unsubstituted (C1-C30)alkyl, a substituted or unsubstituted (C6-C30)aryl, a substituted or unsubstituted 3- to 30-membered heteroaryl, a substituted or unsubstituted (C3-C30)cycloalkyl, a substituted or unsubstituted (C1-C30)alkoxy, a substituted or unsubstituted tri(C1-C30)alkylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted tri(C6-C30)arylsilyl, a substituted or unsubstituted mono- or di- (C1-C30)alkylamino, a substituted or unsubstituted mono- or di- (C6-C30)arylamino, or a substituted or unsubstituted (C1-C30)alkyl(C6-C30)arylamino; or are linked to an adjacent substituent to form a substituted or unsubstituted, mono- or polycyclic, (C3-C30) alicyclic or aromatic ring, whose carbon atom(s) may be replaced with at least one heteroatom selected from nitrogen, oxygen, and sulfur. Ar preferably represents hydrogen, a substituted or unsubstituted (C6-C20)aryl, a substituted or unsubstituted 5- to 20-membered heteroaryl, or a substituted or unsubstituted di(C6-C15)arylamino; and more preferably represents hydrogen, an unsubstituted (C6-C20)aryl, a 5- to 20-membered heteroaryl unsubstituted or substituted with a (C1-C6)alkyl, a (C6-C25)aryl, a 5- to 20-membered heteroaryl, or a (C1-C6)alkyl(C6-C15)aryl, or an unsubstituted di(C6-C15)arylamino. R₁ preferably represents hydrogen, or a substituted or unsubstituted di(C6-C15)arylamino; or is linked to an adjacent substituent to form a substituted or unsubstituted, mono- or polycyclic, (C3-C15) alicyclic or aromatic ring, whose carbon atom(s) may be replaced with at least one heteroatom selected from nitrogen, oxygen, and sulfur; and more preferably represents hydrogen, or a di(C6-C15)arylamino unsubstituted or substituted with a (C1-C6)alkyl; or is linked to an adjacent substituent to form an unsubstituted monocyclic (C3-C15) aromatic ring, for example, a benzene ring.

Specifically, Ar may be a substituted or unsubstituted phenyl, a substituted or unsubstituted naphthyl, a substituted or unsubstituted biphenyl, a substituted or unsubstituted terphenyl, a substituted or unsubstituted triazinyl, a substituted or unsubstituted pyrimidinyl, a substituted or unsubstituted quinazolinyl, a substituted or unsubstituted benzoquinazolinyl, a substituted or unsubstituted quinoxalinyl, a substituted or unsubstituted benzoquinoxalinyl, a substituted or unsubstituted quinolyl, a substituted or unsubstituted benzoquinolyl, a substituted or unsubstituted isoquinolyl, a substituted or unsubstituted benzoisoquinolyl, a substituted or unsubstituted triazolyl, a substituted or unsubstituted pyrazolyl, a substituted or unsubstituted dibenzothiophenyl, a substituted or unsubstituted benzothiophenyl, a substituted or unsubstituted dibenzofuranyl, or a substituted or unsubstituted benzofuranyl. More specifically, Ar may be a triazinyl, pyrimidinyl, quinazolinyl, benzoquinazolinyl, quinoxalinyl, benzoquinoxalinyl, quinolyl, benzoquinolyl, isoquinolyl, benzoisoquinolyl, triazolyl, pyrazolyl, dibenzothiophenyl, benzothiophenyl, dibenzofuranyl, or benzofuranyl, substituted with an aryl or a heteroaryl.

According to one embodiment of the present disclosure, in formula 1 above, X₁ to X₁₀ each represent CR₁; X₁₁ to X₁₃ each independently represent N or CR₁; L represents a single bond, a substituted or unsubstituted (C6-C15)arylene, or a substituted or unsubstituted 5- to 15-membered heteroarylene; Ar represents hydrogen, a substituted or unsubstituted (C6-C20)aryl, a substituted or unsubstituted 5- to 20-membered heteroaryl, or a substituted or unsubstituted di(C6-C15)arylamino; and R₁ represents hydrogen, or a substituted or unsubstituted di(C6-C15)arylamino; or is linked to an adjacent substituent to form a substituted or unsubstituted, mono- or polycyclic, (C3-C15) alicyclic or aromatic ring, whose carbon atom(s) may be replaced with at least one heteroatom selected from nitrogen, oxygen, and sulfur.

According to another embodiment of the present disclosure, in formula 1 above, X₁ to X₁₀ each represent CR₁; X₁₁ to X₁₃ each independently represent N or CR₁; L represents a single bond, an unsubstituted (C6-C15)arylene, or an unsubstituted 5- to 15-membered heteroarylene; Ar represents hydrogen; an unsubstituted (C6-C20)aryl; a 5- to 20-membered heteroaryl unsubstituted or substituted with a (C1-C6)alkyl, a (C6-C25)aryl, a 5-to 20-membered heteroaryl, or a (C1-C6)alkyl(C6-C15)aryl; or an unsubstituted di(C6-C15)arylamino; and R₁ represents hydrogen, or a di(C6-C15)arylamino unsubstituted or substituted with a (C1-C6)alkyl; or is linked to an adjacent substituent to form an unsubstituted monocyclic (C3-C15) aromatic ring.

The organic electroluminescent compound represented by formula 1 includes the following compounds, but is not limited thereto:

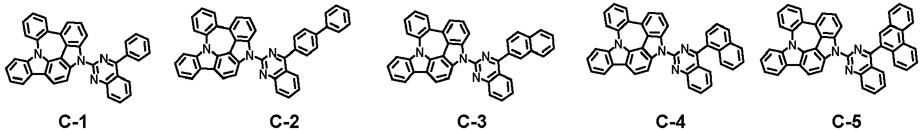

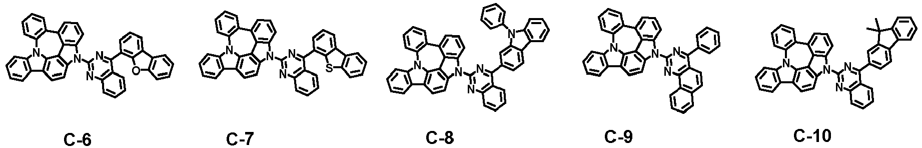

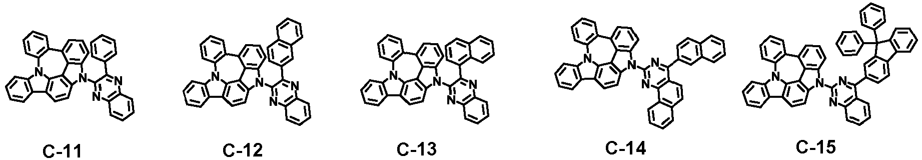

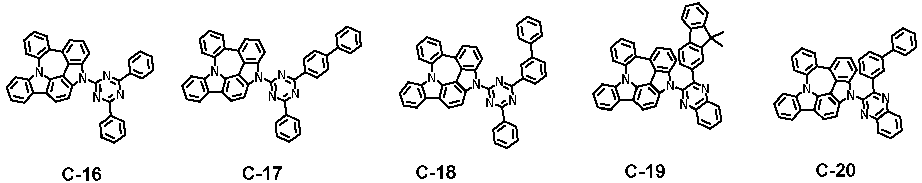

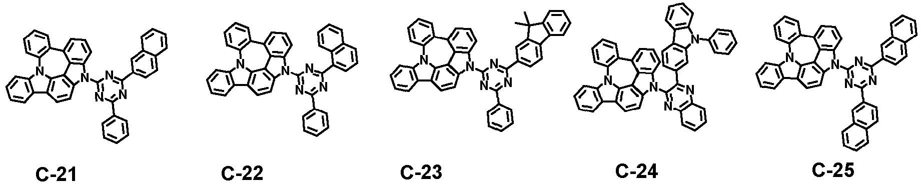

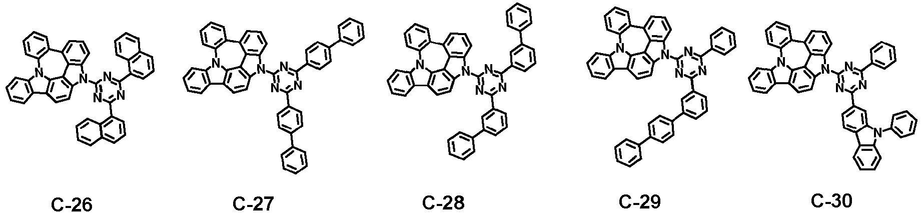

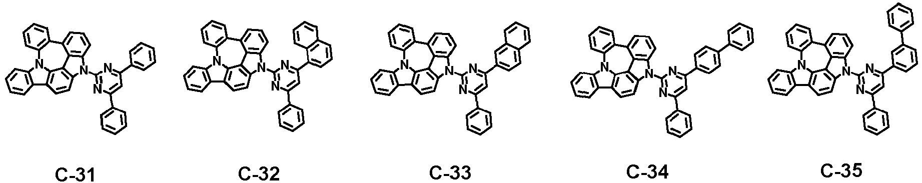

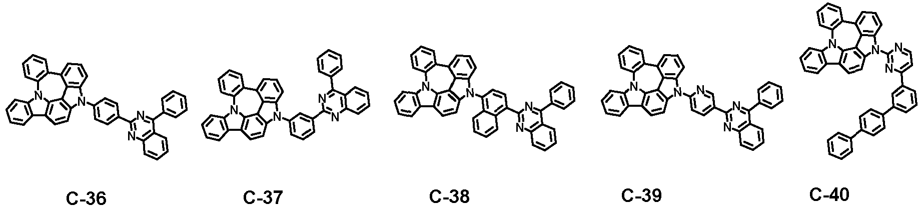

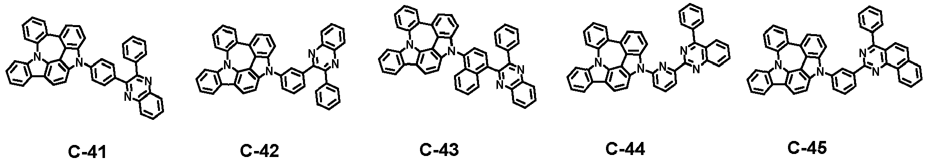

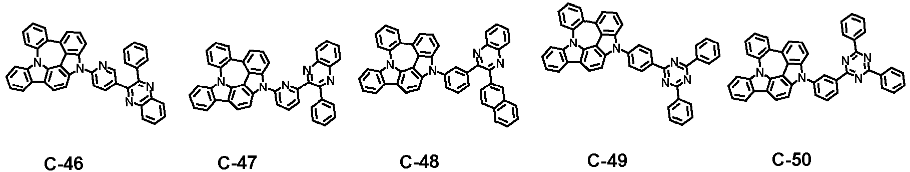

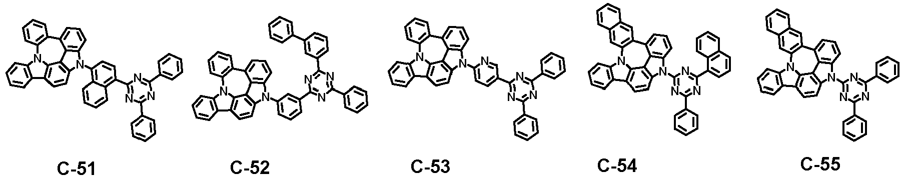

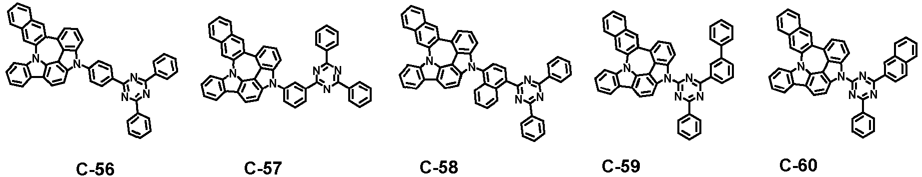

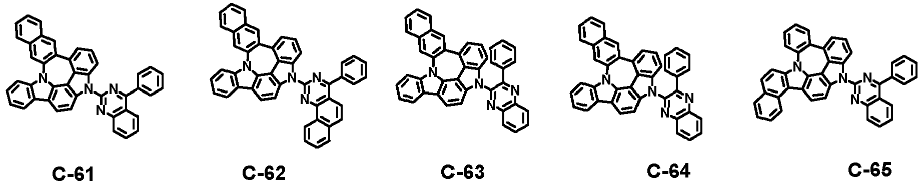

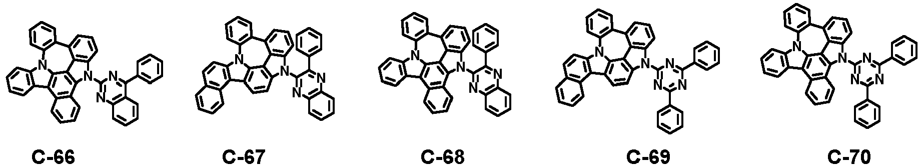

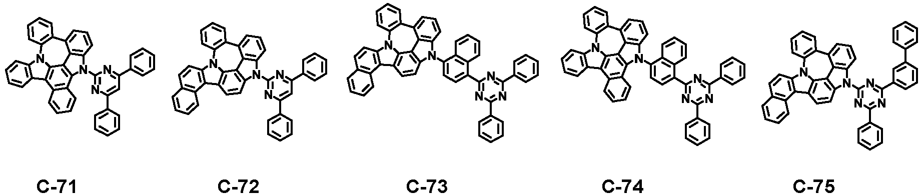

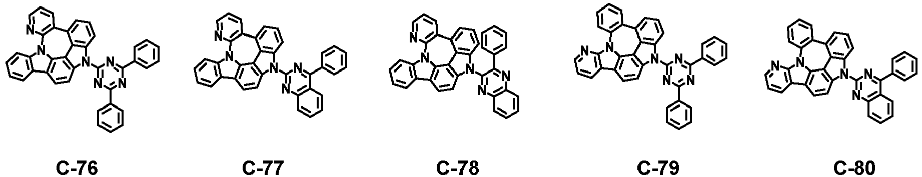

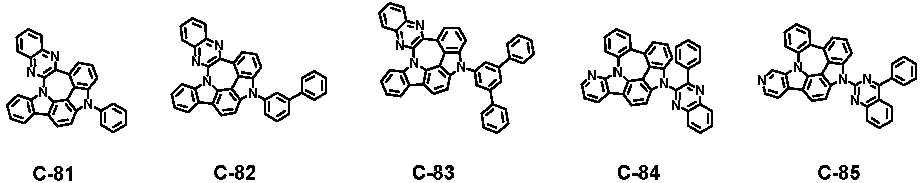

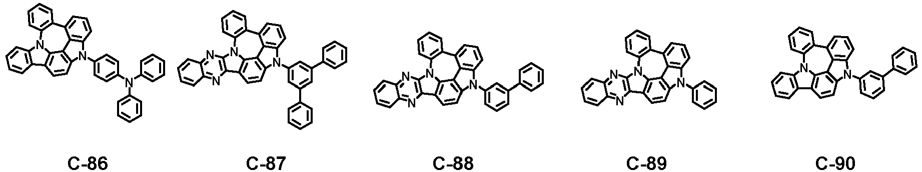

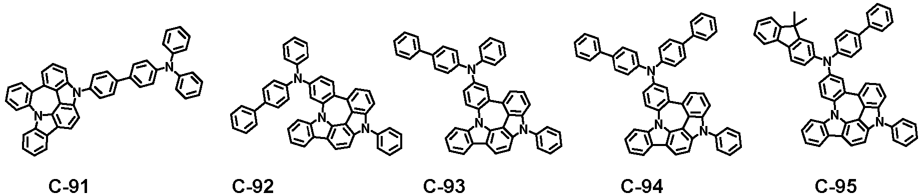

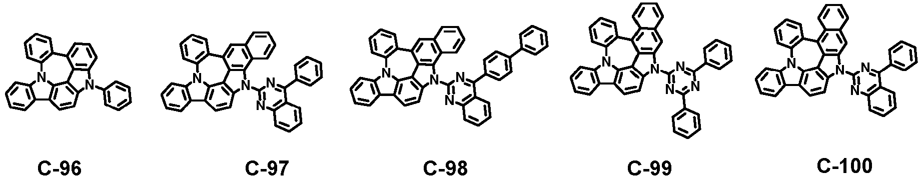

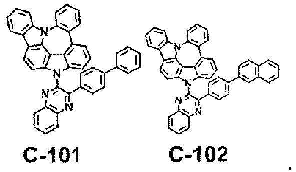

The organic electroluminescent compound according to the present disclosure can be prepared by a synthetic method known to a person skilled in the art. For example, it can be prepared according to the following reaction scheme. 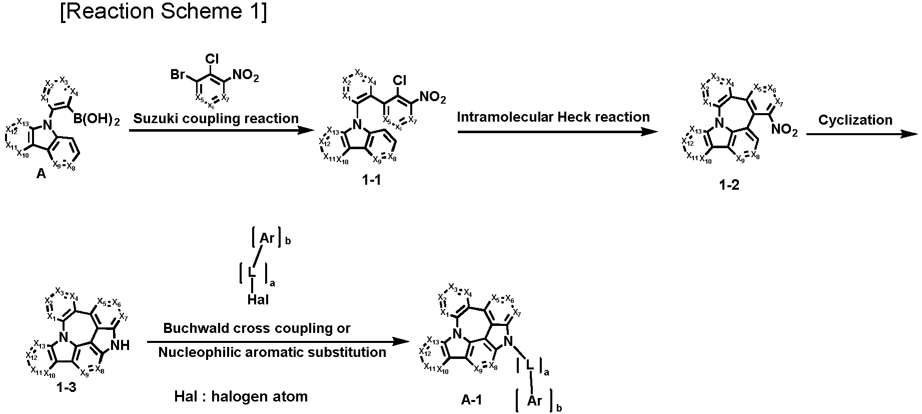 wherein X₁ to X₁₃, L, Ar, R₁, a, and b are as defined in formula 1, and Hal represents a halogen.

The present disclosure provides an organic electroluminescent material comprising the organic electroluminescent compound of formula 1, and an organic electroluminescent device comprising the material.

The above material can be comprised of the organic electroluminescent compound according to the present disclosure alone, or can further include conventional materials generally used in organic electroluminescent materials.

The organic electroluminescent device according to the present disclosure comprises a first electrode; a second electrode; and at least one organic layer between the first and second electrodes. The organic layer may comprise at least one organic electroluminescent compound of formula 1.

One of the first and second electrodes may be an anode, and the other may be a cathode. The organic layer may comprise a light-emitting layer, and may further comprise at least one layer selected from a hole injection layer, a hole transport layer, a hole auxiliary layer, a light-emitting auxiliary layer, an electron transport layer, an electron buffer layer, an electron injection layer, an interlayer, a hole blocking layer, and an electron blocking layer.

The organic electroluminescent compound of formula 1 of the present disclosure may be comprised in the light-emitting layer. Where used in the light-emitting layer, the organic electroluminescent compound of formula 1 of the present disclosure can be comprised as a host material. Preferably, the light-emitting layer can further comprise one or more dopants. If necessary, the organic electroluminescent compound of the present disclosure can be used as a co-host material. That is, the light-emitting layer can additionally comprise a compound other than the organic electroluminescent compound of formula 1 of the present disclosure (first host material) as a second host material. Herein, the weight ratio of the first host material to the second host material is in the range of 1:99 to 99:1.

The second host material can be any of the known hosts. The host selected from the group consisting of the compounds of formulas 11 to 16 below may be preferable in terms of luminous efficiency.

H-(Cz-L₄)ₕ-M ----------- (11)

H-(Cz)ᵢ-L₄-M ----------- (12)

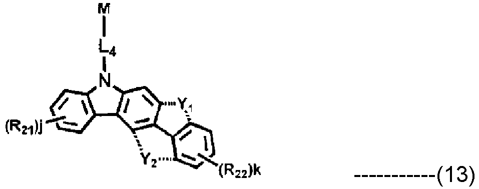

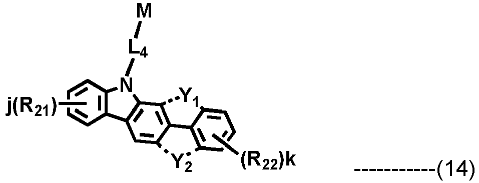

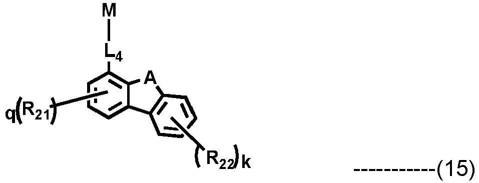

wherein
Cz represents the following structure: 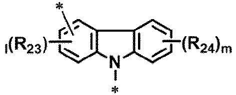
A represents -O- or -S-; and
R₂₁ to R₂₄, each independently, represent hydrogen, deuterium, a halogen, a substituted or unsubstituted (C1-C30)alkyl, a substituted or unsubstituted (C6-C30)aryl, a substituted or unsubstituted 5- to 30-membered heteroaryl, or -SiR₂₅R₂₆R₂₇; in which R₂₅ to R₂₇, each independently, represent a substituted or unsubstituted (C1-C30)alkyl, or a substituted or unsubstituted (C6-C30)aryl; L₄ represents a single bond, a substituted or unsubstituted (C6-C30)arylene, or a substituted or unsubstituted 5- to 30-membered heteroarylene; M represents a substituted or unsubstituted (C6-C30)aryl, or a substituted or unsubstituted 5- to 30-membered heteroaryl; Y₁ and Y₂, each independently, represent -O-, -S-, -N(R₃₁)- or -C(R₃₂)(R₃₃)-, with the proviso that Y₁ and Y₂ are not present simultaneously; R₃₁ to R₃₃, each independently, represent a substituted or unsubstituted (C1-C30)alkyl, a substituted or unsubstituted (C6-C30)aryl, or a substituted or unsubstituted 5- to 30-membered heteroaryl; R₃₂ and R₃₃ may be the same or different; h and i, each independently, represent an integer of 1 to 3; j, k, I, and m, each independently, represent an integer of 0 to 4; q represents an integer of 0 to 3; if h, i, j, k, I, m, or q represents an integer of 2 or more, each (Cz-L₄), each (Cz), each R₂₁, each R₂₂, each R₂₃, or each R₂₄ may be the same or different; 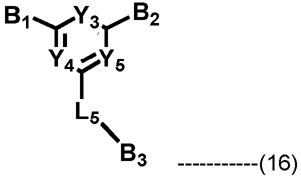
wherein
Y₃ to Y₅, each independently, represent CR₃₄ or N;
R₃₄ represents hydrogen, a substituted or unsubstituted (C1-C30)alkyl, a substituted or unsubstituted (C6-C30)aryl, or a substituted or unsubstituted 5- to 30-membered heteroaryl;
B₁ and B₂, each independently, represent hydrogen, a substituted or unsubstituted (C6-C30)aryl, or a substituted or unsubstituted 5- to 30-membered heteroaryl;
B₃ represents a substituted or unsubstituted (C6-C30)aryl, or a substituted or unsubstituted 5- to 30-membered heteroaryl; and
L₅ represents a single bond, a substituted or unsubstituted (C6-C30)arylene, or a substituted or unsubstituted 5- to 30-membered heteroarylene.

Specifically, the examples of the second host material are as follows, but are not limited thereto. 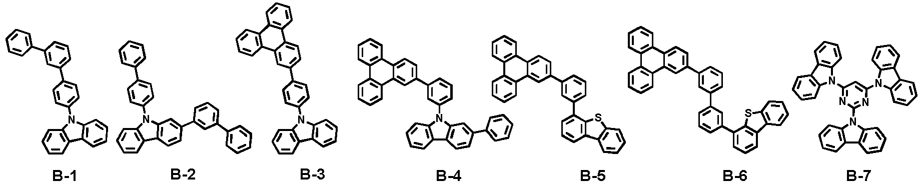 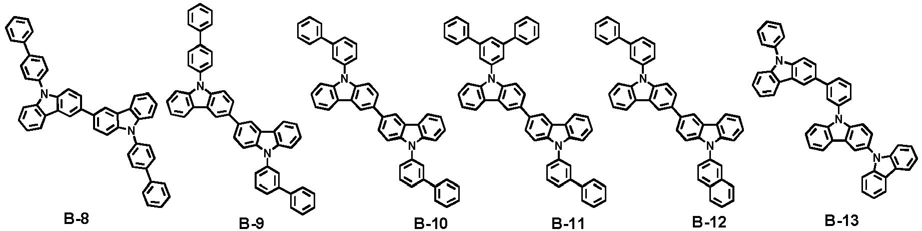 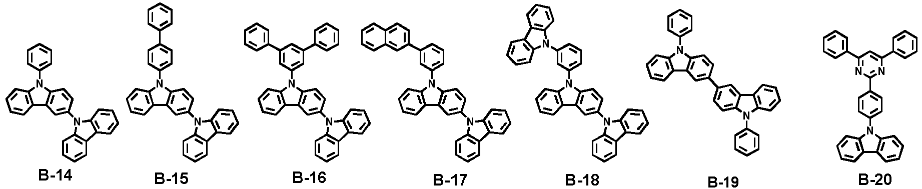 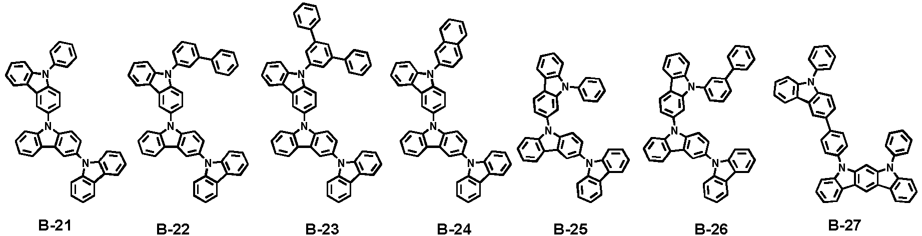 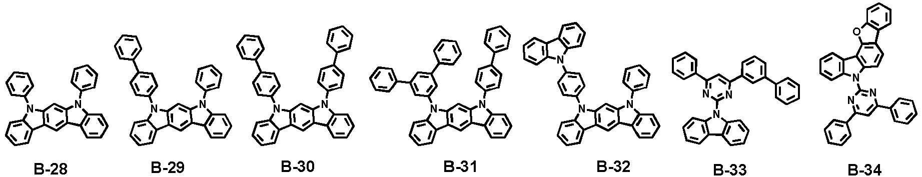 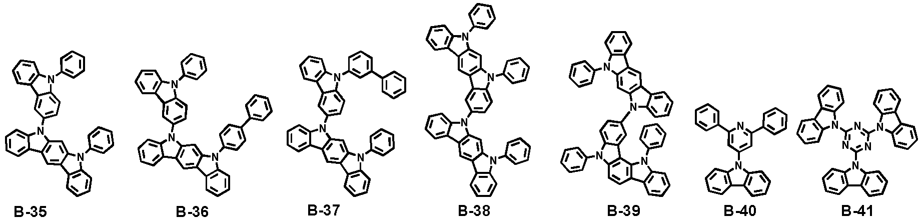 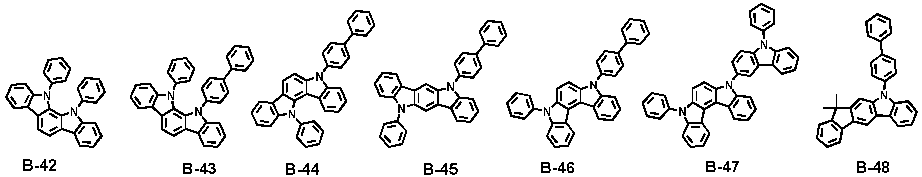 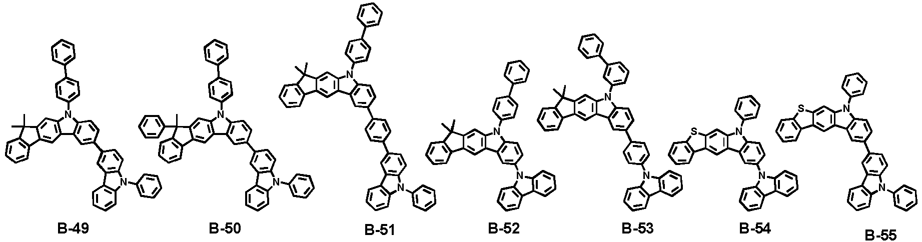 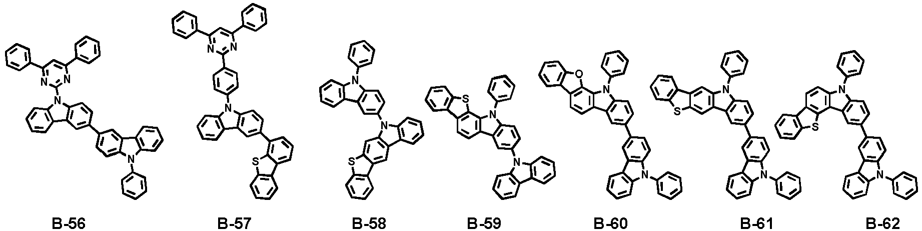 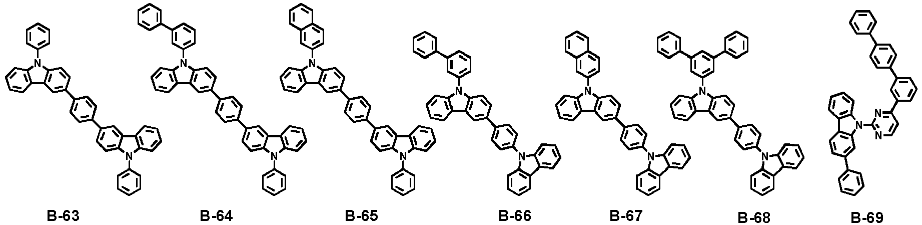 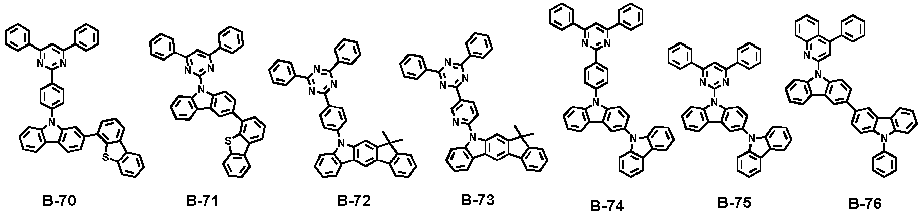 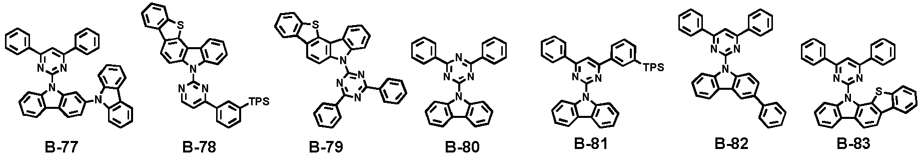 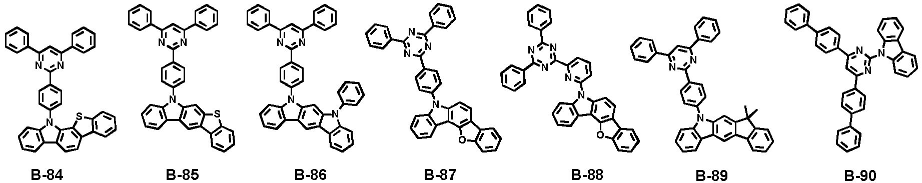 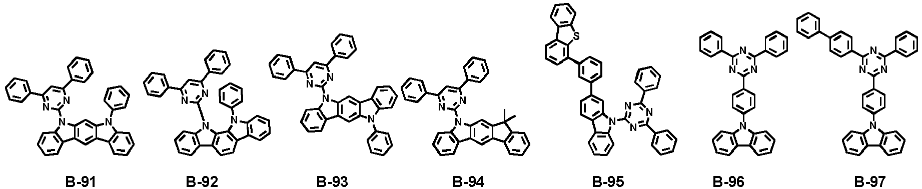 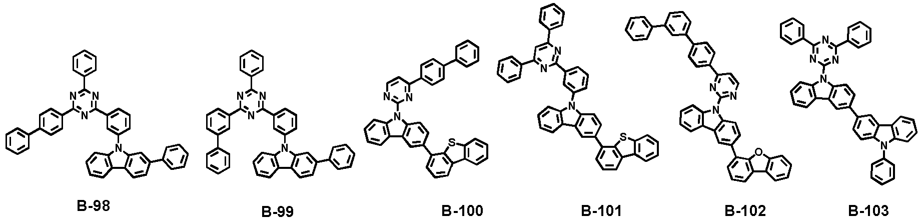 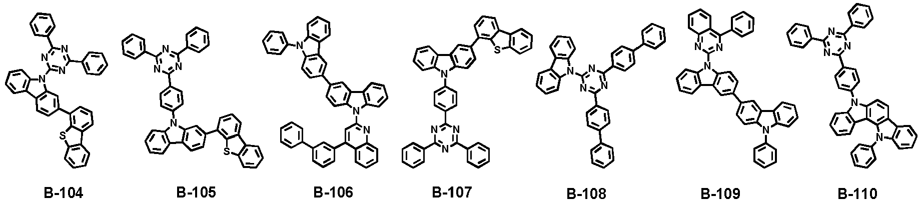 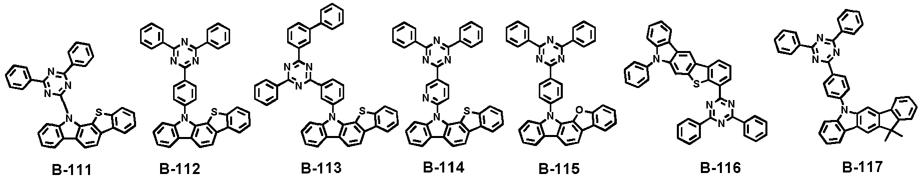 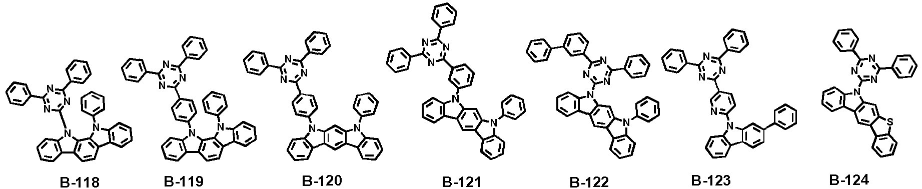 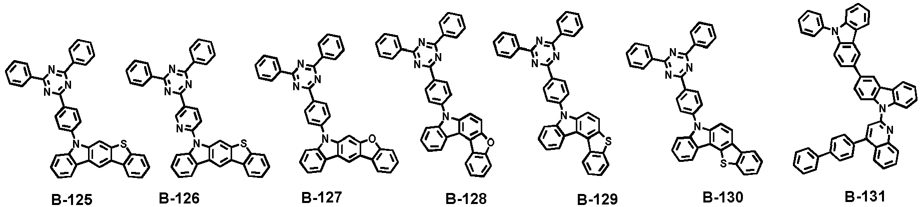 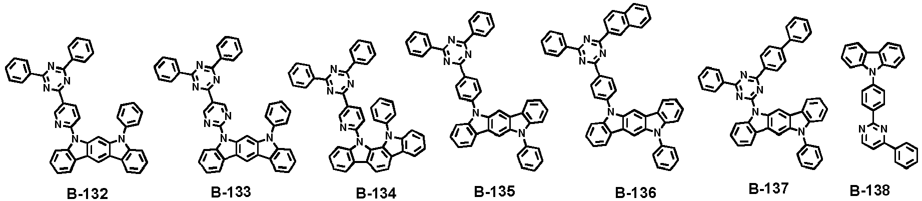 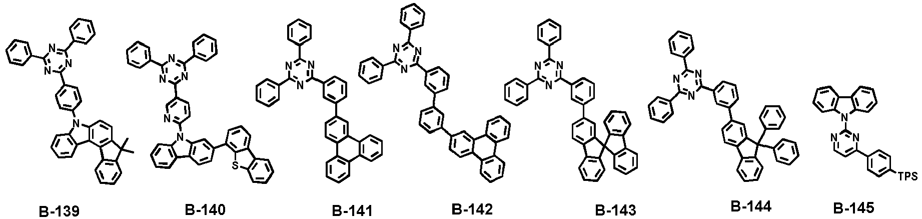 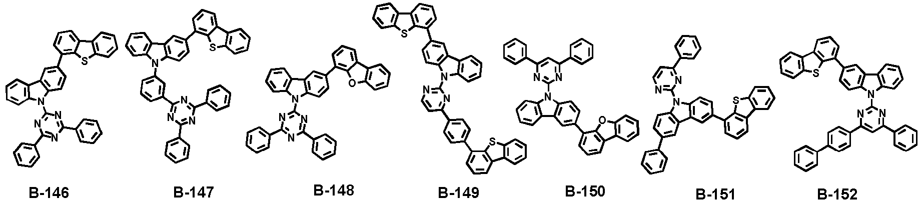 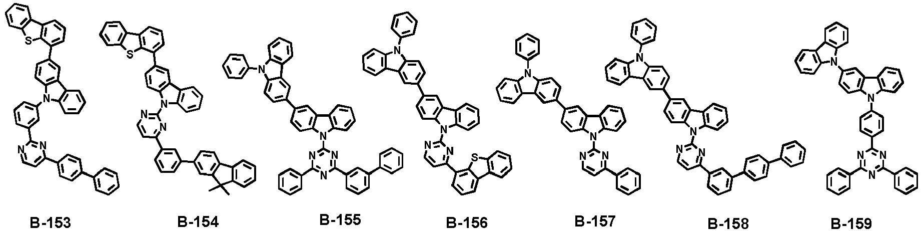 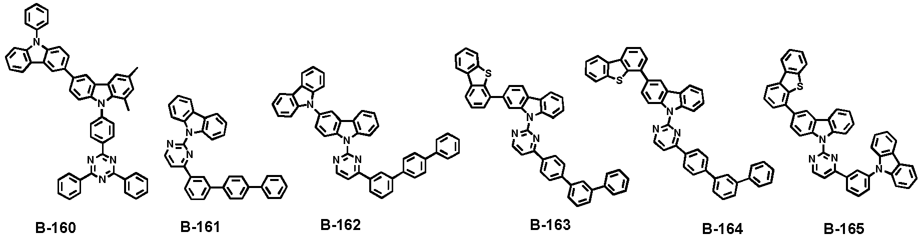 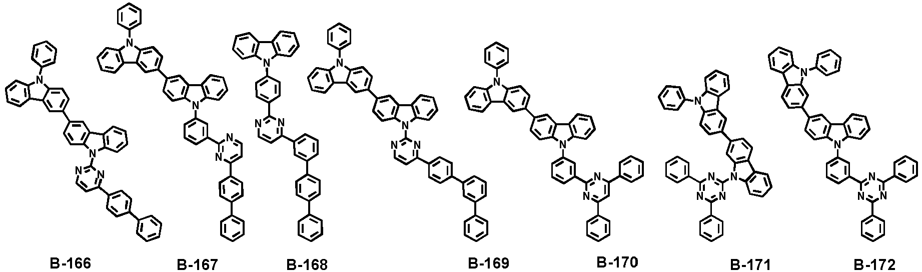 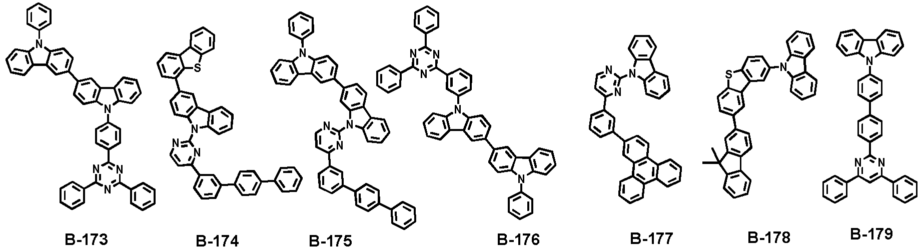 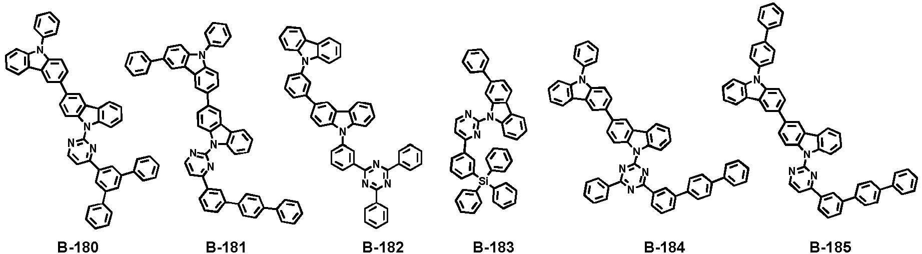 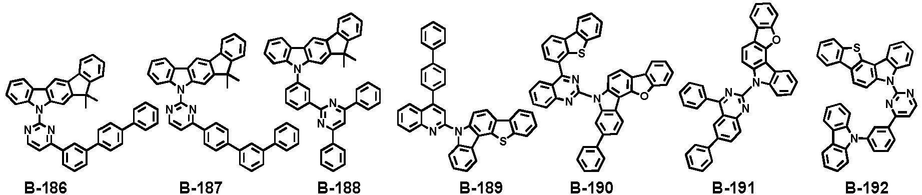 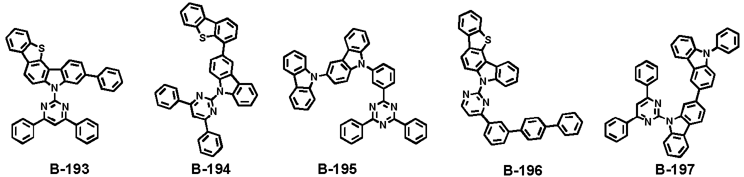 [wherein TPS represents a triphenylsilyl group]

The dopant comprised in the organic electroluminescent device according to the present disclosure may be preferably at least one phosphorescent dopant. The phosphorescent dopant materials applied to the organic electroluminescent device according to the present disclosure are not particularly limited, but may be preferably selected from metallated complex compounds of iridium (Ir), osmium (Os), copper (Cu), and platinum (Pt), may be more preferably selected from ortho-metallated complex compounds of iridium (Ir), osmium (Os), copper (Cu), and platinum (Pt), and may be even more preferably an ortho-metallated iridium complex compound.

The dopant comprised in the organic electroluminescent device of the present disclosure may be selected from the group consisting of the compounds represented by formulas 101 to 104 below, but is not limited thereto. 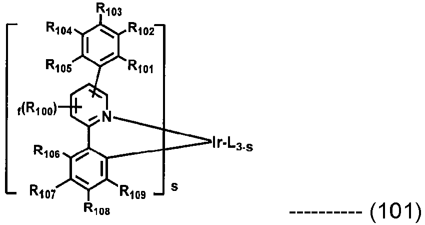 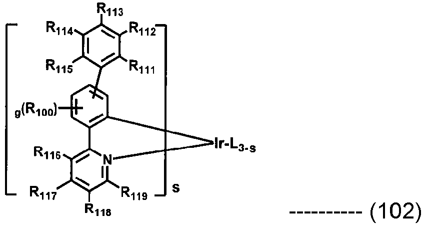 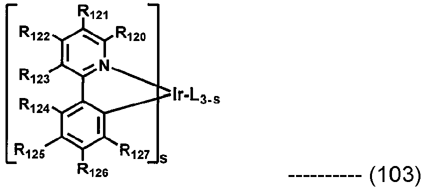 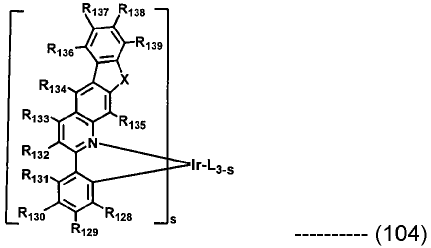 wherein L is selected from the following structures: 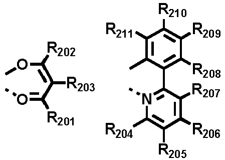
R₁₀₀, R₁₃₄, and R₁₃₅, each independently, represent hydrogen, deuterium, a substituted or unsubstituted (C1-C30)alkyl, or a substituted or unsubstituted (C3-C30)cycloalkyl;
R₁₀₁ to R₁₀₉ and R₁₁₁ to R₁₂₃, each independently, represent hydrogen, deuterium, a halogen, a (C1-C30)alkyl unsubstituted or substituted with deuterium or a halogen, a substituted or unsubstituted (C3-C30)cycloalkyl, a substituted or unsubstituted (C6-C30)aryl, a cyano, or a substituted or unsubstituted (C1-C30)alkoxy; an adjacent substituent of R₁₀₆ to R₁₀₉ may be linked to each other to form a substituted or unsubstituted fused ring, e.g., a fluorene unsubstituted or substituted with an alkyl, a dibenzothiophene unsubstituted or substituted with an alkyl, or a dibenzofuran unsubstituted or substituted with an alkyl; and adjacent substituents of R₁₂₀ to R₁₂₃ may be linked to each other to form a substituted or unsubstituted fused ring, e.g., a quinoline unsubstituted or substituted with at least one of an alkyl, an aryl, an aralkyl, and an alkylaryl;
R₁₂₄ to R₁₃₃ and R₁₃₆ to R₁₃₉, each independently, represent hydrogen, deuterium, a halogen, a substituted or unsubstituted (C1-C30)alkyl, or a substituted or unsubstituted (C6-C30)aryl; and adjacent substituents of R₁₂₄ to R₁₂₇ may be linked to each other to form a substituted or unsubstituted fused ring, e.g., a fluorene unsubstituted or substituted with an alkyl, a dibenzothiophene unsubstituted or substituted with an alkyl, or a dibenzofuran unsubstituted or substituted with an alkyl;
X represents CR₂₁ R₂₂, O, or S;
R₂₁ and R₂₂, each independently, represent a substituted or unsubstituted (C1-C10)alkyl, or a substituted or unsubstituted (C6-C30)aryl;
R₂₀₁ to R₂₁₁, each independently, represent hydrogen, deuterium, a halogen, a (C1-C30)alkyl unsubstituted or substituted with deuterium or a halogen, a substituted or unsubstituted (C3-C30)cycloalkyl, or a (C6-C30)aryl unsubstituted or substituted with an alkyl or deuterium; and adjacent substituents of R₂₀₈ to R₂₁₁ may be linked to each other to form a substituted or unsubstituted fused ring, e.g., a fluorene unsubstituted or substituted with an alkyl, a dibenzothiophene unsubstituted or substituted with an alkyl, or a dibenzofuran unsubstituted or substituted with an alkyl;
f and g, each independently, represent an integer of 1 to 3; where f or g is an integer of 2 or more, each R₁₀₀ may be the same or different; and
s represents an integer of 1 to 3.

The specific examples of the dopant compound are as follows:

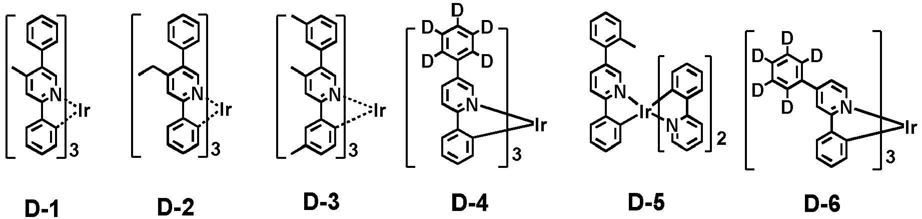

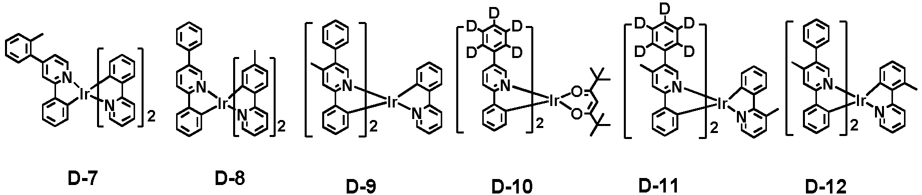

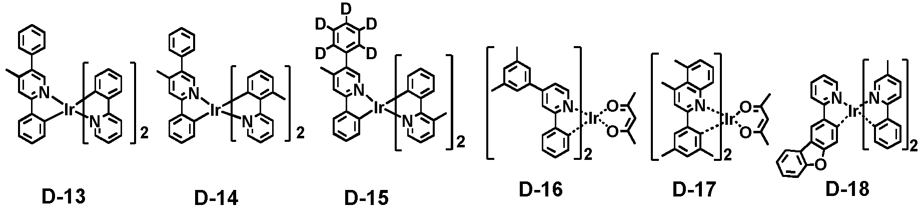

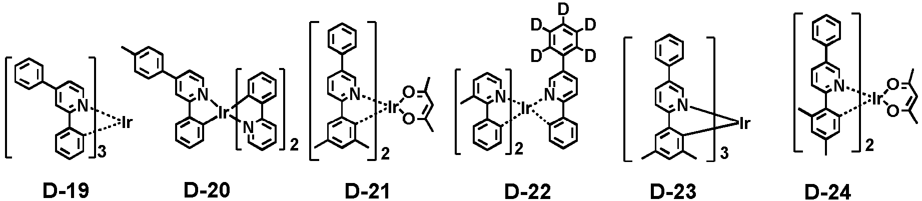

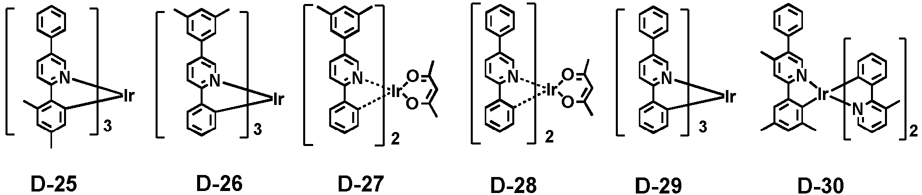

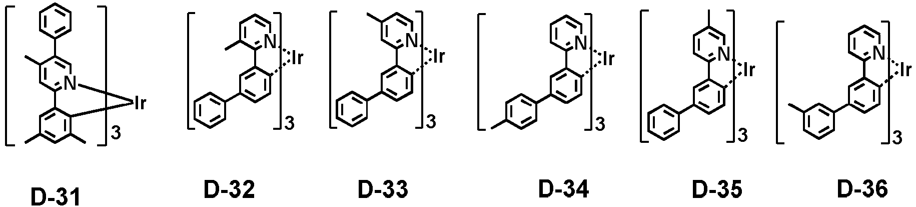

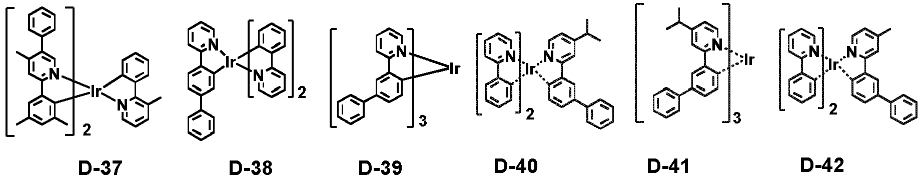

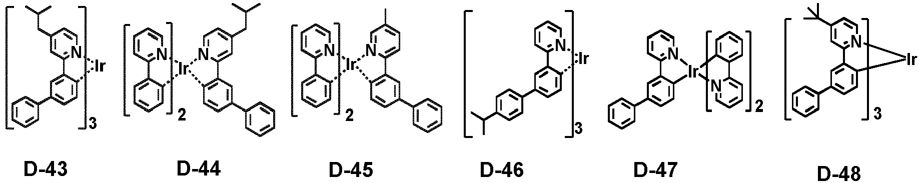

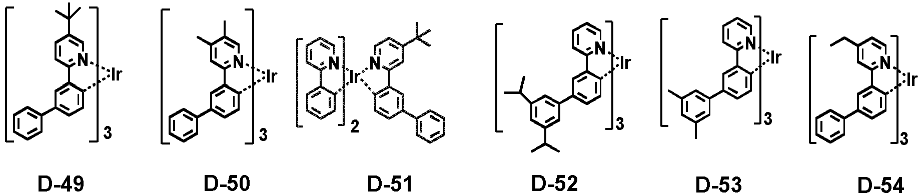

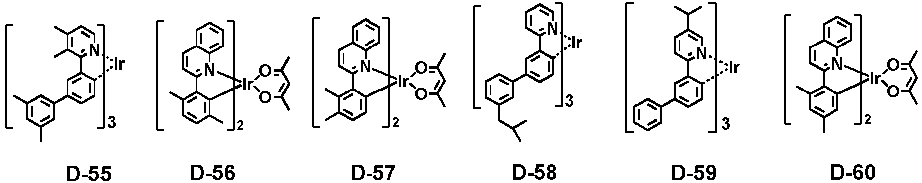

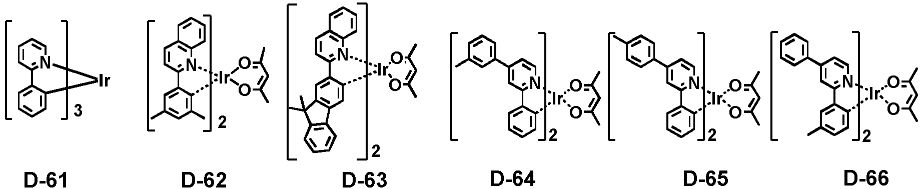

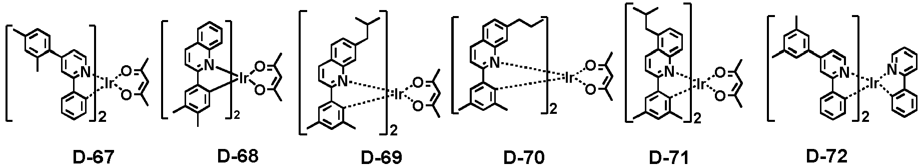

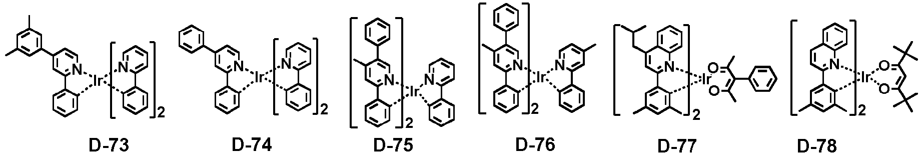

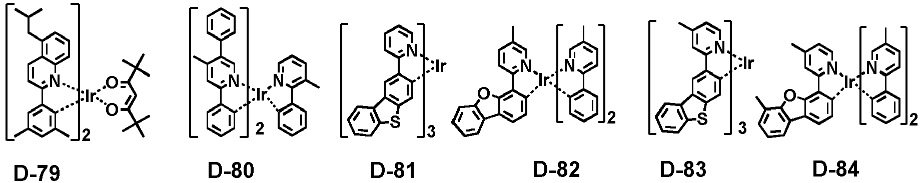

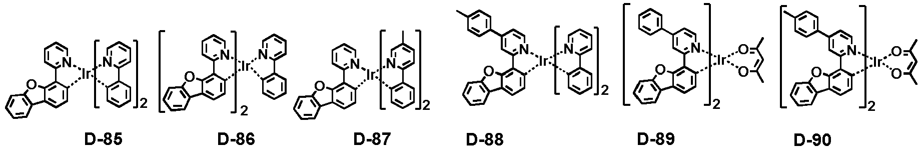

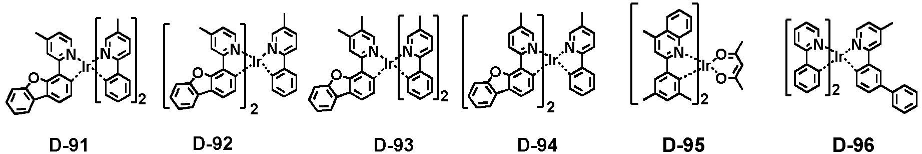

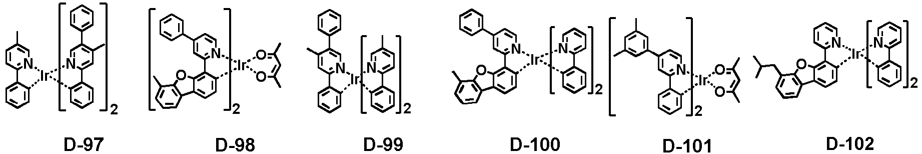

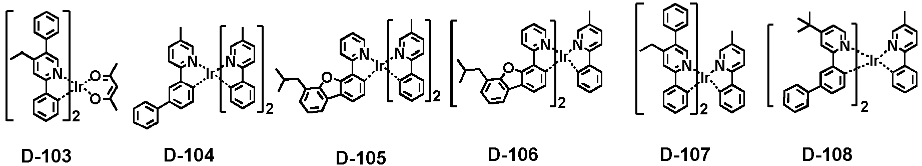

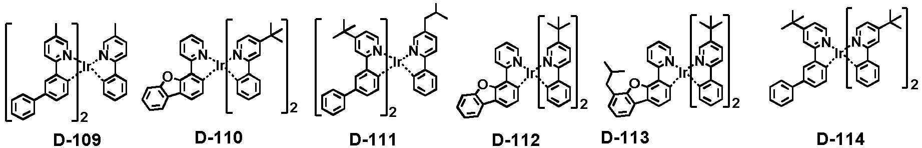

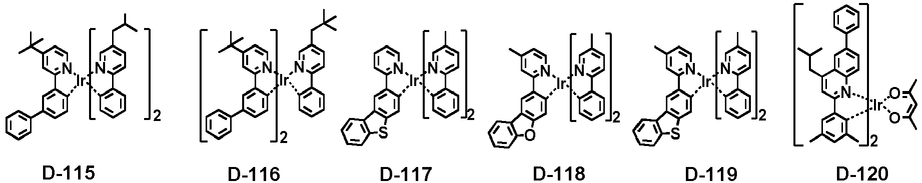

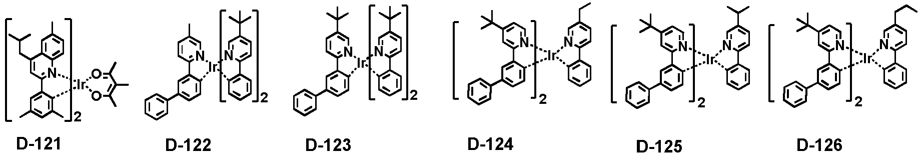

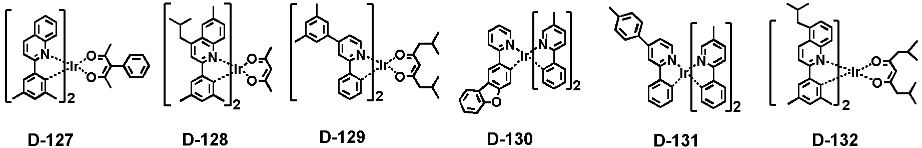

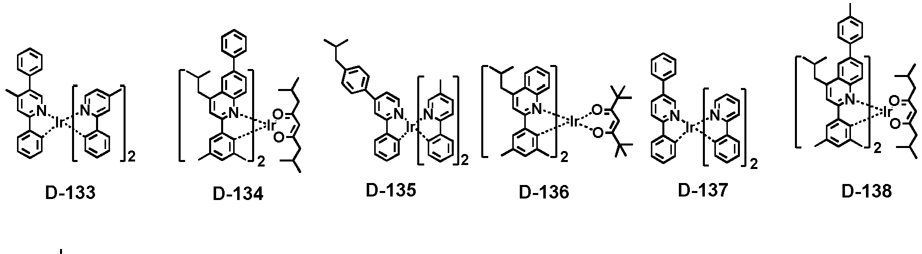

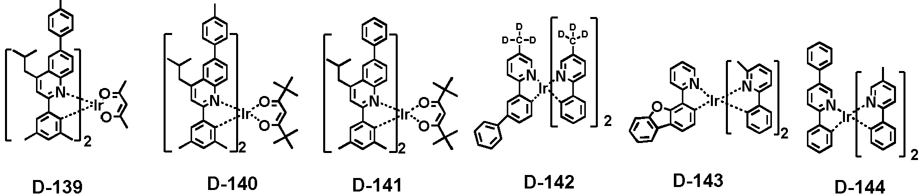

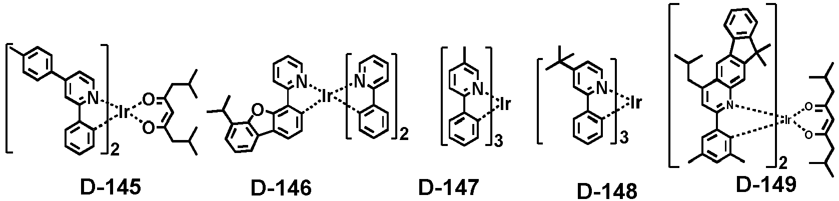

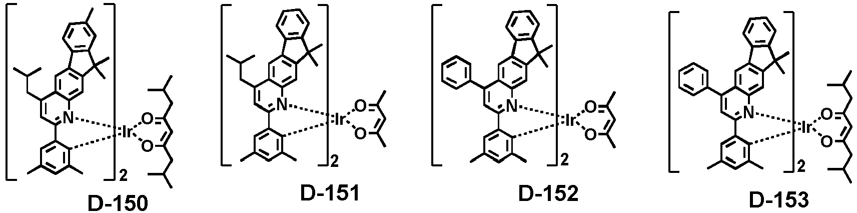

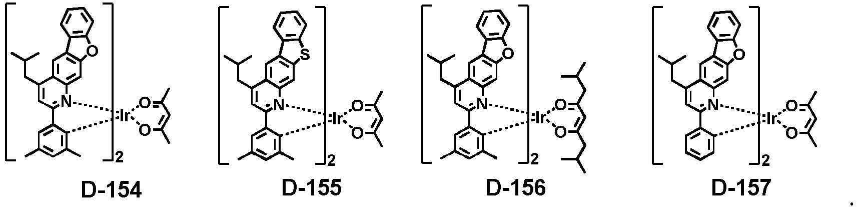

In another embodiment of the present disclosure, a composition for preparing an organic electroluminescent device is provided. The composition comprises the compound according to the present disclosure as a host material, a hole transport layer material, a hole auxiliary layer material, a light-emitting auxiliary layer material, an electron buffer layer material, or an electron transport layer material.

In addition, the organic electroluminescent device according to the present disclosure comprises a first electrode; a second electrode; and at least one organic layer between the first and second electrodes. The organic layer comprises a light-emitting layer, and the light-emitting layer may comprise the composition for preparing the organic electroluminescent device according to the present disclosure.

The organic electroluminescent device according to the present disclosure may further comprise, in addition to the organic electroluminescent compound of formula 1, at least one compound selected from the group consisting of arylamine-based compounds and styrylarylamine-based compounds.

In the organic electroluminescent device according to the present disclosure, the organic layer may further comprise, in addition to the organic electroluminescent compound of formula 1, at least one metal selected from the group consisting of metals of Group 1, metals of Group 2, transition metals of the 4^{th} period, transition metals of the 5^{th} period, lanthanides and organic metals of d-transition elements of the Periodic Table, or at least one complex compound comprising said metal. The organic layer may further comprise a light-emitting layer and a charge generating layer.

In addition, the organic electroluminescent device according to the present disclosure may emit white light by further comprising at least one light-emitting layer which comprises a blue electroluminescent compound, a red electroluminescent compound or a green electroluminescent compound known in the field, besides the compound according to the present disclosure. Also, if necessary, a yellow or orange light-emitting layer can be further comprised in the device.

In the organic electroluminescent device according to the present disclosure, at least one layer (hereinafter, "a surface layer") is preferably placed on an inner surface(s) of one or both electrode(s); selected from a chalcogenide layer, a metal halide layer, and a metal oxide layer. Specifically, a chalcogenide (including oxides) layer of silicon or aluminum is preferably placed on an anode surface of an electroluminescent medium layer, and a metal halide layer or a metal oxide layer is preferably placed on a cathode surface of an electroluminescent medium layer. Such a surface layer provides operation stability for the organic electroluminescent device. Preferably, said chalcogenide includes SiOₓ(1≤X≤2), AlOₓ(1≤X≤1.5), SiON, SiAlON, etc.; said metal halide includes LiF, MgF₂, CaF₂, a rare earth metal fluoride, etc.; and said metal oxide includes Cs₂O, Li₂O, MgO, SrO, BaO, CaO, etc.

A hole injection layer, a hole transport layer, an electron blocking layer, or a combination thereof can be used between the anode and the light-emitting layer. The hole injection layer may be multi-layers in order to lower the hole injection barrier (or hole injection voltage) from the anode to the hole transport layer or the electron blocking layer, wherein each of the multi-layers may use two compounds simultaneously. The hole transport layer or the electron blocking layer may also be multi-layers.

An electron buffer layer, a hole blocking layer, an electron transport layer, an electron injection layer, or a combination thereof can be used between the light-emitting layer and the cathode. The electron buffer layer may be multi-layers in order to control the injection of the electron and improve the interfacial properties between the light-emitting layer and the electron injection layer, wherein each of the multi-layers may use two compounds simultaneously. The hole blocking layer or the electron transport layer may also be multi-layers, wherein each of the multi-layers may use a plurality of compounds.

The light-emitting auxiliary layer may be placed between the anode and the light-emitting layer, or between the cathode and the light-emitting layer. When the light-emitting auxiliary layer is placed between the anode and the light-emitting layer, it can be used for promoting the hole injection and/or hole transport, or for preventing the overflow of electrons. When the light-emitting auxiliary layer is placed between the cathode and the light-emitting layer, it can be used for promoting the electron injection and/or electron transport, or for preventing the overflow of holes. Also, the hole auxiliary layer may be placed between the hole transport layer (or hole injection layer) and the light-emitting layer, and may be effective to promote or block the hole transport rate (or hole injection rate), thereby enabling the charge balance to be controlled. Further, the electron blocking layer may be placed between the hole transport layer (or hole injection layer) and the light-emitting layer, and can confine the excitons within the light-emitting layer by blocking the overflow of electrons from the light-emitting layer to prevent a light-emitting leakage. When an organic electroluminescent device includes two or more hole transport layers, the hole transport layer, which is further included, may be used as a hole auxiliary layer or an electron blocking layer. The hole auxiliary layer and the electron blocking layer may have an effect of improving the efficiency and/or the lifespan of the organic electroluminescent device.

Preferably, in the organic electroluminescent device of the present disclosure, a mixed region of an electron transport compound and a reductive dopant, or a mixed region of a hole transport compound and an oxidative dopant may be placed on at least one surface of a pair of electrodes. In this case, the electron transport compound is reduced to an anion, and thus it becomes easier to inject and transport electrons from the mixed region to the light-emitting medium. Furthermore, the hole transport compound is oxidized to a cation, and thus it becomes easier to inject and transport holes from the mixed region to the light-emitting medium. Preferably, the oxidative dopant includes various Lewis acids and acceptor compounds; and the reductive dopant includes alkali metals, alkali metal compounds, alkaline earth metals, rare-earth metals, and mixtures thereof. The reductive dopant layer may be employed as a charge-generating layer to prepare an organic EL device having two or more light-emitting layers which emits white light.

In order to form each layer constituting the organic electroluminescent device of the present disclosure, dry film-forming methods such as vacuum deposition, sputtering, plasma, ion plating methods, etc., or wet film-forming methods such as spin coating, dip coating, flow coating methods, etc., can be used. When forming the film of the first and second host compounds of the present disclosure, a co-evaporation or a mixed evaporation method is used.

When using a wet film-forming method, a thin film is formed by dissolving or dispersing the material constituting each layer in suitable solvents, such as ethanol, chloroform, tetrahydrofuran, dioxane, etc. The solvents are not specifically limited as long as the material constituting each layer is soluble or dispersible in the solvents, which do not cause any problems in forming a film.

By using the organic electroluminescent device of the present disclosure, a display device, for example, for smartphones, tablets, notebooks, PCs, TVs, or vehicles, or a lighting device, for example, an indoor or outdoor lighting device, can be produced.

Hereinafter, the preparation method of the organic electroluminescent compounds of the present disclosure, the physical properties of the compounds, and the luminous properties of the organic electroluminescent device comprising the compounds will be explained in detail with reference to the representative compounds of the present disclosure.

### Example 1: Preparation of compound C-50

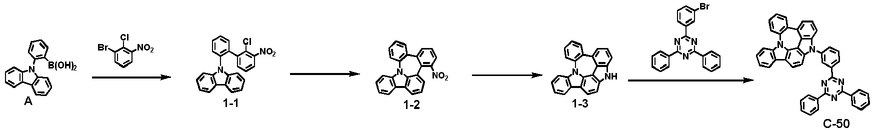

### Preparation of compound 1-1

36 g of compound **A** (125.38 mmol), 27 g of 3-bromo-2-chloro-nitrobenzene (113.98 mmol), 4 g of tetrakis(triphenylphosphine)palladium (3.42 mmol), 30 g of sodium carbonate (284.95 mmol), 570 mL of toluene, 140 mL of ethanol, and 140 mL of distilled water were introduced into a reaction vessel, and the mixture was stirred at 120°C for 3 hours. After completion of the reaction, the mixture was cooled to room temperature and extracted with ethyl acetate. The extracted organic layer was then dried with magnesium sulfate, and the solvent was removed with a rotary evaporator. Thereafter, the resulting product was purified by column chromatography to obtain 30 g of compound **1-1** (yield: 66%).

### Preparation of compound 1-2

27 g of compound **1-1** (68.20 mmol), 1.5 g of palladium (II) acetate (6.82 mmol), 5.0 g of tricyclohexylphosphoniumtetrafluoroborate (13.64 mmol), 66 g of cesium carbonate (204.60 mmol), and 340 mL of o-xylene were introduced into a reaction vessel, and the mixture was stirred under reflux for 2 hours. After completion of the reaction, the mixture was washed with distilled water, extracted with ethyl acetate, and the extracted organic layer was dried with magnesium sulfate. The solvent was removed with a rotary evaporator, and the resulting product was purified by column chromatography to obtain 26 g of compound **1-2.**

### Preparation of compound 1-3

Compound **1-2** (68.20 mmol) and 176 mL of triethylphosphite (0.4 M) were introduced into a reaction vessel, and the mixture was stirred at 150°C for 4 hours. After completion of the reaction, triethylphosphite was removed by distillation under reduced pressure. The mixture was then washed with distilled water, extracted with ethyl acetate, and the extracted organic layer was dried with magnesium sulfate. The solvent was removed with a rotary evaporator, and the resulting product was purified by column chromatography to obtain 16.4 g of compound **1-3** (yield: 70%).

### Preparation of compound C-50

7 g of compound **1-3** (21.19 mmol), 9.9 g of 2-(3-bromophenyl)-4,6-diphenyl-1,3,5-triazine (25.43 mmol), 0.5 g of palladium (II) acetate (2.12 mmol), 1.8 g of 2-dicyclohexylphosphino-2',6'-dimethoxybiphenyl (s-phos) (4.24 mmol), 3.1 g of sodium tert-butoxide (31.79 mmol), and 110 mL of o-xylene were introduced into a reaction vessel, and the mixture was stirred under reflux for 3 hours. After completion of the reaction, the mixture was washed with distilled water, extracted with ethyl acetate, and the extracted organic layer was dried with magnesium sulfate. The solvent was removed with a rotary evaporator, and the resulting product was purified by column chromatography to obtain 5 g of compound **C-50** (yield: 37%).

| | MW | UV | PL | M.P. |
|---|---|---|---|---|
| C-50 | 637.73 | 324 nm | 499 nm | 291°C |

### Example 2: Preparation of compound C-51

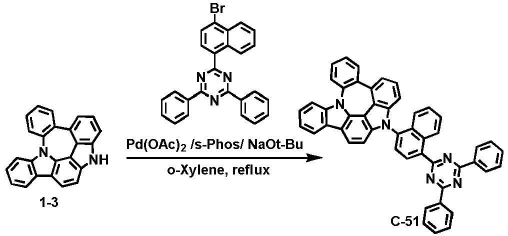

5 g of compound **1-3** (15 mmol), 8.6 g of 2-(4-bromonaphthylen-1-yl)-4,6-diphenyl-1,3,5-triazine (20 mmol), 0.4 g of palladium (II) acetate (2 mmol), 1.2 g of s-phos (3 mmol), 2.2 g of sodium tert-butoxide (23 mmol), and 76 mL of o-xylene were introduced into a reaction vessel, and the mixture was stirred under reflux for 3 hours. After completion of the reaction, the mixture was washed with distilled water, extracted with ethyl acetate, and the extracted organic layer was dried with magnesium sulfate. The solvent was removed with a rotary evaporator, and the resulting product was purified by column chromatography to obtain 3.3 g of compound **C-51** (yield: 32%).

| | MW | UV | PL | M.P. |
|---|---|---|---|---|
| C-51 | 687.79 | 424 nm | 497 nm | 303°C |

### Example 3: Preparation of compound C-49

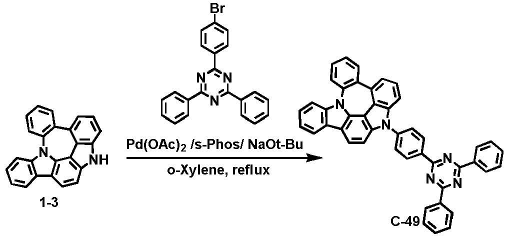

6 g of compound **1-3** (18 mmol), 8.5 g of 2-(4-bromophenyl)-4,6-diphenyl-1,3,5-triazine (22 mmol), 0.4 g of palladium (II) acetate (2 mmol), 1.5 g of s-phos (4 mmol), 2.6 g of sodium tert-butoxide (27 mmol), and 91 mL of o-xylene were introduced into a reaction vessel, and the mixture was stirred under reflux for 2 hours. After completion of the reaction, the mixture was washed with distilled water, extracted with ethyl acetate, and the extracted organic layer was dried with magnesium sulfate. The solvent was removed with a rotary evaporator, and the resulting product was purified by column chromatography to obtain 10 g of compound **C-49** (yield: 86%).

| | MW | M.P. |
|---|---|---|
| C-49 | 637.75 | 274°C |

### Example 4: Preparation of compound C-17

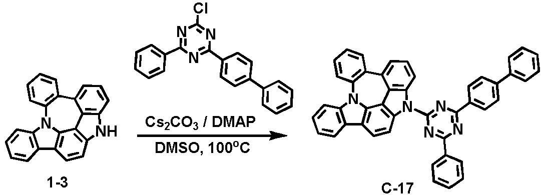

6 g of compound **1-3** (18 mmol), 7.5 g of 2-([1,1'-biphenyl]-4-yl)-4-chloro-6-phenyl-1,3,5-triazine (21 mmol), 5.9 g of cesium carbonate (18 mmol), 1.1 g of 4-dimethylaminopyridine (DMAP) (9 mmol), and 90 mL of dimethylsulfoxide (DMSO) were introduced into a reaction vessel, and the mixture was stirred at 100°C for 3 hours. After completion of the reaction, the mixture was washed with distilled water, extracted with ethyl acetate, and the extracted organic layer was dried with magnesium sulfate. The solvent was removed with a rotary evaporator, and the resulting product was purified by column chromatography to obtain 10.5 g of compound **C-17** (yield: 91%).

| | MW | UV | PL | M.P. |
|---|---|---|---|---|
| C-17 | 637.73 | 392 nm | 497 nm | 240°C |

### Example 5: Preparation of compound C-21

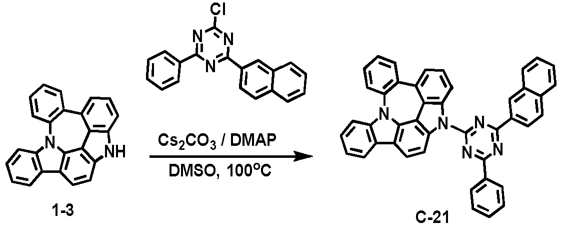

6 g of compound **1-3** (18 mmol), 6.9 g of 2-chloro-4-(naphthylen-2-yl)-6-phenyl-1,3,5-triazine (21 mmol), 5.9 g of cesium carbonate (18 mmol), 1.1 g of DMAP (9 mmol), and 90 mL of DMSO were introduced into a reaction vessel, and the mixture was stirred at 100°C for 3 hours. After completion of the reaction, the mixture was washed with distilled water, extracted with ethyl acetate, and the extracted organic layer was dried with magnesium sulfate. The solvent was removed with a rotary evaporator, and the resulting product was purified by column chromatography to obtain 10 g of compound **C-21** (yield: 90%).

| | MW | UV | PL | M.P. |
|---|---|---|---|---|
| C-21 | 611.69 | 392 nm | 499 nm | 274°C |

### Example 6: Preparation of compound C-11

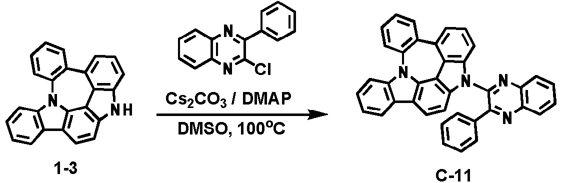

5 g of compound **1-3** (15 mmol), 4.4 g of 2-chloro-3-phenylquinoxaline (18 mmol), 4.9 g of cesium carbonate (15 mmol), 0.9 g of DMAP (8 mmol), and 76 mL of DMSO were introduced into a reaction vessel, and the mixture was stirred at 100°C for 3 hours. After completion of the reaction, the mixture was washed with distilled water, extracted with ethyl acetate, and the extracted organic layer was dried with magnesium sulfate. The solvent was removed with a rotary evaporator, and the resulting product was purified by column chromatography to obtain 1.8 g of compound **C-11** (yield: 22%).

| | MW | UV | PL | M.P. |
|---|---|---|---|---|
| C-11 | 534.62 | 332 nm | 529 nm | 180°C |

### Example 7: Preparation of compound C-12

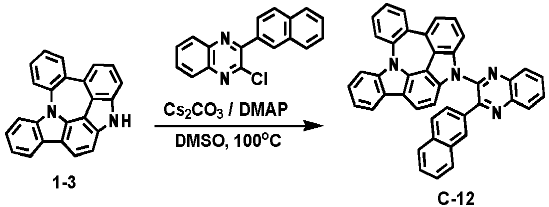

5 g of compound **1-3** (15 mmol), 5.2 g of 2-chloro-3-naphthylquinoxaline (18 mmol), 4.9 g of cesium carbonate (15 mmol), 0.9 g of DMAP (8 mmol), and 76 mL of DMSO were introduced into a reaction vessel, and the mixture was stirred at 100°C for 3 hours. After completion of the reaction, the mixture was washed with distilled water, extracted with ethyl acetate, and the extracted organic layer was dried with magnesium sulfate. The solvent was removed with a rotary evaporator, and the resulting product was purified by column chromatography to obtain 4.2 g of compound **C-12** (yield: 48%).

| | MW | UV | PL | M.P. |
|---|---|---|---|---|
| C-12 | 584.68 | 324 nm | 525 nm | 170°C |

### Example 8: Preparation of compound C-67

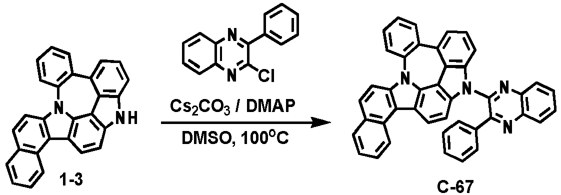

2.7 g of compound **1-3** (7 mmol), 1.0 g of 2-chloro-3-phenylquinoxaline (4 mmol), 2.3 g of cesium carbonate (7 mmol), 0.5 g of DMAP (4 mmol), and 36 mL of DMSO were introduced into a reaction vessel, and the mixture was stirred at 100°C for 5 hours. After completion of the reaction, the mixture was washed with distilled water, extracted with ethyl acetate, and the extracted organic layer was dried with magnesium sulfate. The solvent was removed with a rotary evaporator, and the resulting product was purified by column chromatography to obtain 1 g of compound **C-67** (yield: 40%).

| | MW | M.P. |
|---|---|---|
| C-67 | 584.67 | 285°C |

### Example 9: Preparation of compound C-1

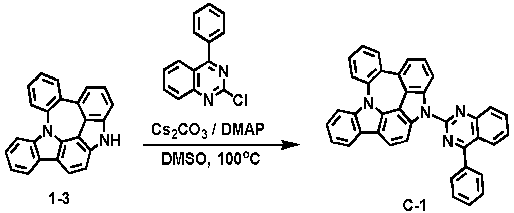

4 g of compound **1-3** (12 mmol), 3.8 g of 2-chloro-4-phenylquinazoline (16 mmol), 3.9 g of cesium carbonate (12 mmol), 0.7 g of DMAP (6 mmol), and 60 mL of DMSO were introduced into a reaction vessel, and the mixture was stirred at 100°C for 3 hours. After completion of the reaction, the mixture was washed with distilled water, extracted with ethyl acetate, and the extracted organic layer was dried with magnesium sulfate. The solvent was removed with a rotary evaporator, and the resulting product was purified by column chromatography to obtain 5.4 g of compound **C-1** (yield: 83%).

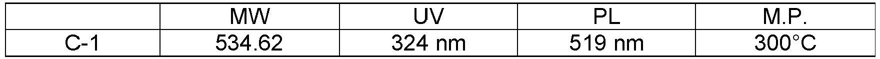

### Example 10: Preparation of compound C-101

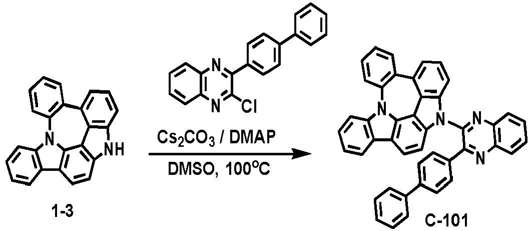

5 g of compound **1-3** (15 mmol), 5.7 g of 2-([1,1'-biphenyl]-4-yl)-3-chloroquinoxaline (18 mmol), 4.9 g of cesium carbonate (15 mmol), 0.9 g of DMAP (8 mmol), and 76 mL of DMSO were introduced into a reaction vessel, and the mixture was stirred at 100°C for 3 hours. After completion of the reaction, the mixture was washed with distilled water, extracted with ethyl acetate, and the extracted organic layer was dried with magnesium sulfate. The solvent was removed with a rotary evaporator, and the resulting product was purified by column chromatography to obtain 5.0 g of compound **C-101** (yield: 55%).

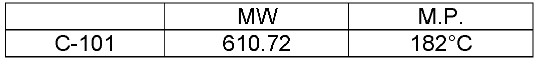

### Example 11: Preparation of compound C-102

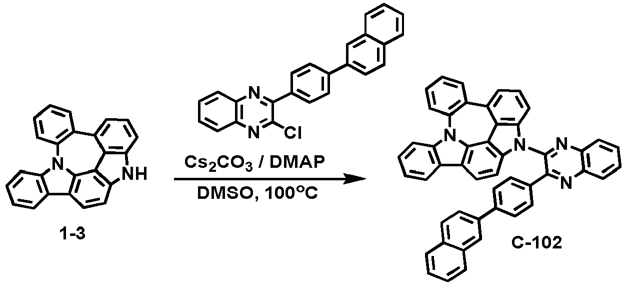

3 g of compound **1-3** (9 mmol), 4.0 g of 2-chloro-3-(4-(naphthalen-2-yl)phenyl)quinoxaline (11 mmol), 2.9 g of cesium carbonate (9 mmol), 0.4 g of DMAP (4 mmol), and 45 mL of DMSO were introduced into a reaction vessel, and the mixture was stirred at 100°C for 5 hours. After completion of the reaction, the mixture was washed with distilled water, extracted with ethyl acetate, and the extracted organic layer was dried with magnesium sulfate. The solvent was removed with a rotary evaporator, and the resulting product was purified by column chromatography to obtain 1.8 g of compound **C-102** (yield: 31%).

| | MW | M.P. |
|---|---|---|
| C-102 | 660.78 | 186°C |

### Device Example 1: Production of an OLED device using the organic electroluminescent compound according to the present disclosure

An OLED device was produced using the organic electroluminescent compound of the present disclosure. A transparent electrode indium tin oxide (ITO) thin film (10 Ω/sq) on a glass substrate for an organic light-emitting diode (OLED) device (Geomatec, Japan) was subjected to an ultrasonic washing with acetone, ethanol, and distilled water, sequentially, and was then stored in isopropanol. Next, the ITO substrate was mounted on a substrate holder of a vacuum vapor depositing apparatus. Compound HI-1 was introduced into a cell of said vacuum vapor depositing apparatus, and then the pressure in the chamber of said apparatus was controlled to 10⁻⁶ torr. Thereafter, an electric current was applied to the cell to evaporate the above-introduced material, thereby forming a first hole injection layer having a thickness of 80 nm on the ITO substrate. Compound HI-2 was then introduced into another cell of said vacuum vapor depositing apparatus, and was evaporated by applying an electric current to the cell, thereby forming a second hole injection layer having a thickness of 5 nm on the first hole injection layer. Compound HT-1 was introduced into another cell of said vacuum vapor depositing apparatus, and was evaporated by applying an electric current to the cell, thereby forming a first hole transport layer having a thickness of 10 nm on the second hole injection layer. Compound HT-3 was introduced into another cell of said vacuum vapor depositing apparatus, and was evaporated by applying an electric current to the cell, thereby forming a second hole transport layer having a thickness of 60 nm on the first hole transport layer. After forming the hole injection layers and the hole transport layers, a light-emitting layer was then deposited as follows. Compound C-50 was introduced into one cell of the vacuum vapor depositing apparatus as a host, and compound D-71 was introduced into another cell as a dopant. The two materials were evaporated and were deposited in a doping amount of 3 wt% based on the total amount of the host and dopant to form a light-emitting layer having a thickness of 40 nm on the second hole transport layer. Compound ET-1 and compound EI-1 were then introduced into other two cells, evaporated at the rate of 1:1, and deposited to form an electron transport layer having a thickness of 30 nm on the light-emitting layer. Next, after depositing compound EI-1 as an electron injection layer having a thickness of 2 nm on the electron transport layer, an Al cathode having a thickness of 80 nm was deposited by another vacuum vapor deposition apparatus on the electron injection layer. Thus, an OLED device was produced.

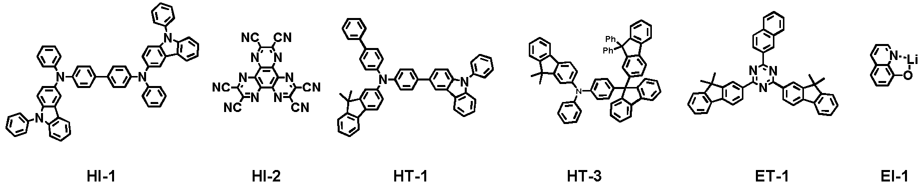

As a result, an efficiency of 28.6 cd/A at 3.5 V was shown, red light of 1000 cd/m² was emitted, and the least time taken to be reduced from 100% to 95% of the luminance at 5,000 nits was 95 hours.

### Comparative Example 1: Production of an OLED device using a conventional organic electroluminescent compound

An OLED device was produced in the same manner as in Device Example 1, except that compound B was used instead of compound C-50.

As a result, an efficiency of 14.3 cd/A at 10 V was shown, red light of 1000 cd/m² was emitted, and the least time taken to be reduced from 100% to 95% of the luminance at 5,000 nits was 0.1 hour.

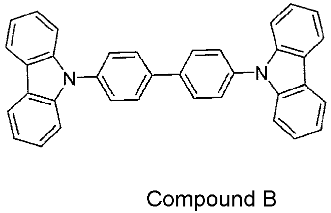

### Comparative Example 2: Production of an OLED device using a conventional organic electroluminescent compound

An OLED device was produced in the same manner as in Device Example 1, except that compound Y was used instead of compound C-50.

As a result, an efficiency of 26.2 cd/A at 4.0 V was shown, red light of 1000 cd/m² was emitted, and the least time taken to be reduced from 100% to 95% of the luminance at 5,000 nits was 34 hours.

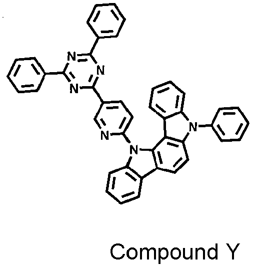

### Comparative Example 3: Production of an OLED device not according to the present disclosure and emitting red light

An OLED device was produced in the same manner as in Device Example 1, except that 4,4'-N,N'-dicarbazol-biphenyl (CBP) was used as a host material instead of compound C-50, compound HT-4 was used as a second hole transport layer material instead of compound HT-3, and the thickness of the electron transport layer was changed to 35 nm.

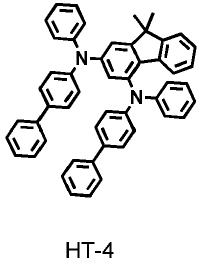

### Device Examples 2 to 11: Production of OLED devices according to the present disclosure and emitting red light

OLED devices were produced in the same manner as in Comparative Example 3, except that the host was changed to the compounds shown in Table 1 below.

The driving voltage, luminous efficiency, and CIE color coordinates at 1,000 nits, and the lifespan (T95) measured as the time taken to be reduced from 100% to 95% of the luminance at 5,000 nits of the OLED devices produced in Comparative Example 3 and Device Examples 2 to 11 are provided in Table 1 below.

**[Table 1]**

| | Host | Driving voltage [V] | Luminous efficiency (cd/A) | Color coordinate (x) | Color coordinate (y) | Lifespan (T95, hr) |
|---|---|---|---|---|---|---|
| Comparative Example 3 | CBP | 9.2 | 9.2 | 0.663 | 0.334 | 0.24 |
| Device Example 2 | C-51 | 3.1 | 25.7 | 0.673 | 0.326 | 196.7 |
| Device Example 3 | C-49 | 3.4 | 27.5 | 0.672 | 0.328 | 167.5 |
| Device Example 4 | C-17 | 3.5 | 26.5 | 0.673 | 0.327 | 261.6 |
| Device Example 5 | C-21 | 3.6 | 25.4 | 0.674 | 0.330 | 189.3 |
| Device Example 6 | C-11 | 3.4 | 29.7 | 0.671 | 0.329 | 163.3 |
| Device Example 7 | C-12 | 3.2 | 28.0 | 0.674 | 0.326 | 50.7 |
| Device Example 8 | C-67 | 3.2 | 26.6 | 0.671 | 0.329 | 204.3 |
| Device Example 9 | C-1 | 3.3 | 25.0 | 0.668 | 0.332 | 350.0 |
| Device Example 10 | C-101 | 3.2 | 26.4 | 0.671 | 0.328 | 173.4 |
| Device Example 11 | C-102 | 3.2 | 25.5 | 0.672 | 0.328 | 162.0 |

When producing organic electroluminescent devices, the organic electroluminescent compounds according to the present disclosure provide lower driving voltage, higher luminous efficiency such as current efficiency, and longer operational lifespan of the devices compared to the conventional organic electroluminescent compounds.

The compound of formula 1 of the present disclosure has an azepine ring formed by a link of a benzene ring, which is substituted on N in an indolocarbazole backbone, and a benzene ring of a carbazole. Such effects of the compound of the present disclosure may be interpreted by the following theory.

Figure 1 shows that the compound of the present disclosure has a reduced steric hindrance compared to the conventional compound. The steric hindrance increases the intermolecular distance, and thus the electron mobility is lowered in electron hopping. Therefore, a hopping distance should be reduced by increasing the current enabling electron hopping. As a result, the driving voltage of the organic electroluminescent device increases. In Figure 1, since an H-H distance in compound A is too short, N-phenyls are not located on the same plane and are vertically located. Thus, the aforementioned steric hindrance is expected. In order to prevent such phenomenon, compound B of the present disclosure was invented to have a planar structure compared to compound A. Accordingly, the compound of the present disclosure can reduce driving voltage upon applying it to a device.

## Claims

1. An organic electroluminescent compound represented by the following formula 1: 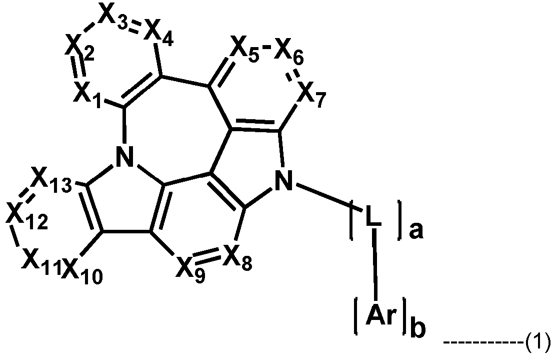 wherein
X₁ to X₁₀ each represent CR₁;
X₁₁ to X₁₃ each independently represent N or CR₁;
L represents a single bond, a substituted or unsubstituted (C6-C30)arylene, or a substituted or unsubstituted 3- to 30-membered heteroarylene;
Ar and R₁ each independently represent hydrogen, deuterium, a halogen, a cyano, a substituted or unsubstituted (C1-C30)alkyl, a substituted or unsubstituted (C6-C30)aryl, a substituted or unsubstituted 3- to 30-membered heteroaryl, a substituted or unsubstituted (C3-C30)cycloalkyl, a substituted or unsubstituted (C1-C30)alkoxy, a substituted or unsubstituted tri(C1-C30)alkylsilyl, a substituted or unsubstituted di(C1-C30)alkyl(C6-C30)arylsilyl, a substituted or unsubstituted (C1-C30)alkyldi(C6-C30)arylsilyl, a substituted or unsubstituted tri(C6-C30)arylsilyl, a substituted or unsubstituted mono- or di- (C1-C30)alkylamino, a substituted or unsubstituted mono- or di- (C6-C30)arylamino, or a substituted or unsubstituted (C1-C30)alkyl(C6-C30)arylamino; or are linked to an adjacent substituent to form a substituted or unsubstituted, mono- or polycyclic, (C3-C30) alicyclic or aromatic ring, whose carbon atom(s) may be replaced with at least one heteroatom selected from nitrogen, oxygen, and sulfur;
the heteroaryl(ene) contains at least one heteroatom selected from B, N, O, S, Si, and P; and
a and b each independently represent an integer of 1 to 2.

2. The organic electroluminescent compound according to claim 1, wherein the substituents of the substituted (C1-C30)alkyl(ene), the substituted (C6-C30)aryl(ene), the substituted 3- to 30-membered heteroaryl(ene), the substituted (C3-C30)cycloalkyl(ene), the substituted (C1-C30)alkoxy, the substituted tri(C1-C30)alkylsilyl, the substituted di(C1-C30)alkyl(C6-C30)arylsilyl, the substituted (C1-C30)alkyldi(C6-C30)arylsilyl, the substituted tri(C6-C30)arylsilyl, the substituted mono- or di- (C1-C30)alkylamino, the substituted mono-or di- (C6-C30)arylamino, the substituted (C1-C30)alkyl(C6-C30)arylamino, and the substituted mono- or polycyclic, (C3-C30) alicyclic or aromatic ring in Ar, L, and R₁ each independently are at least one selected from the group consisting of deuterium, a halogen, a cyano, a carboxyl, a nitro, a hydroxyl, a (C1-C30)alkyl, a halo(C1-C30)alkyl, a (C2-C30)alkenyl, a (C2-C30)alkynyl, a (C1-C30)alkoxy, a (C1-C30)alkylthio, a (C3-C30)cycloalkyl, a (C3-C30)cycloalkenyl, a 3- to 7-membered heterocycloalkyl, a (C6-C30)aryloxy, a (C6-C30)arylthio, a 5- to 30-membered heteroaryl unsubstituted or substituted with a (C6-C30)aryl, a (C6-C30)aryl unsubstituted or substituted with a 5- to 30-membered heteroaryl, a tri(C1-C30)alkylsilyl, a tri(C6-C30)arylsilyl, a di(C1-C30)alkyl(C6-C30)arylsilyl, a (C1-C30)alkyldi(C6-C30)arylsilyl, an amino, a mono- or di- (C1-C30)alkylamino, a mono- or di- (C6-C30)arylamino unsubstituted or substituted with a (C1-C30)alkyl, a (C1-C30)alkyl(C6-C30)arylamino, a (C1-C30)alkylcarbonyl, a (C1-C30)alkoxycarbonyl, a (C6-C30)arylcarbonyl, a di(C6-C30)arylboronyl, a di(C1-C30)alkylboronyl, a (C1-C30)alkyl(C6-C30)arylboronyl, a (C6-C30)aryl(C1-C30)alkyl, and a (C1-C30)alkyl(C6-C30)aryl.

3. The organic electroluminescent compound according to claim 1, wherein formula 1 is represented by any one of the following formulas 2 to 7: 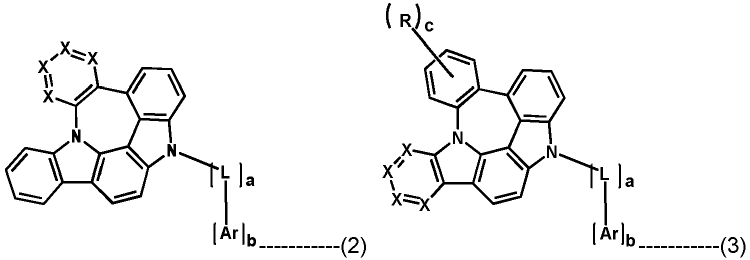 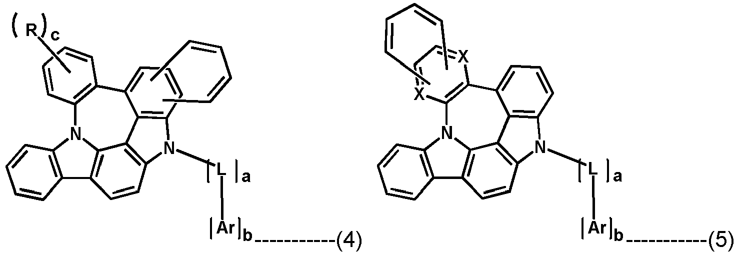 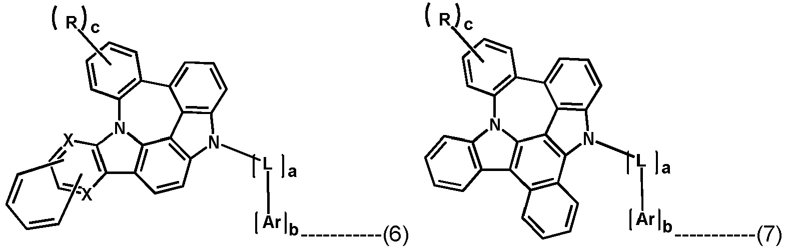 wherein
X represents CR₁;
R represents a substituted or unsubstituted mono- or di- (C6-C30)arylamino;
c represents an integer of 1 to 2; and
L, Ar, R₁, a, and b are as defined in claim 1.

4. The organic electroluminescent compound according to claim 1, wherein
X₁ to X₁₀ each represent CR₁;
X₁₁ to X₁₃ each independently represent N or CR₁;
L represents a single bond, a substituted or unsubstituted (C6-C15)arylene, or a substituted or unsubstituted 5- to 15-membered heteroarylene;
Ar represents hydrogen, a substituted or unsubstituted (C6-C20)aryl, a substituted or unsubstituted 5- to 20-membered heteroaryl, or a substituted or unsubstituted di(C6-C15)arylamino; and
R₁ represents hydrogen, or a substituted or unsubstituted di(C6-C15)arylamino; or is linked to an adjacent substituent to form a substituted or unsubstituted, mono- or polycyclic, (C3-C15) alicyclic or aromatic ring, whose carbon atom(s) may be replaced with at least one heteroatom selected from nitrogen, oxygen, and sulfur.

5. The organic electroluminescent compound according to claim 1, wherein
X₁ to X₁₀ each represent CR₁;
X₁₁ to X₁₃ each independently represent N or CR₁;
L represents a single bond, an unsubstituted (C6-C15)arylene, or an unsubstituted 5- to 15-membered heteroarylene;
Ar represents hydrogen; an unsubstituted (C6-C20)aryl; a 5- to 20-membered heteroaryl unsubstituted or substituted with a (C1-C6)alkyl, a (C6-C25)aryl, a 5- to 20-membered heteroaryl, or a (C1-C6)alkyl(C6-C15)aryl; or an unsubstituted di(C6-C15)arylamino; and
R₁ represents hydrogen, or a di(C6-C15)arylamino unsubstituted or substituted with a (C1-C6)alkyl; or is linked to an adjacent substituent to form an unsubstituted monocyclic (C3-C15) aromatic ring.

6. The organic electroluminescent compound according to claim 1, wherein Ar is a substituted or unsubstituted phenyl, a substituted or unsubstituted naphthyl, a substituted or unsubstituted biphenyl, a substituted or unsubstituted terphenyl, a substituted or unsubstituted triazinyl, a substituted or unsubstituted pyrimidinyl, a substituted or unsubstituted quinazolinyl, a substituted or unsubstituted benzoquinazolinyl, a substituted or unsubstituted quinoxalinyl, a substituted or unsubstituted benzoquinoxalinyl, a substituted or unsubstituted quinolyl, a substituted or unsubstituted benzoquinolyl, a substituted or unsubstituted isoquinolyl, a substituted or unsubstituted benzoisoquinolyl, a substituted or unsubstituted triazolyl, a substituted or unsubstituted pyrazolyl, a substituted or unsubstituted dibenzothiophenyl, a substituted or unsubstituted benzothiophenyl, a substituted or unsubstituted dibenzofuranyl, or a substituted or unsubstituted benzofuranyl.

7. The organic electroluminescent compound according to claim 1, wherein the compound represented by formula 1 is selected from the group consisting of:

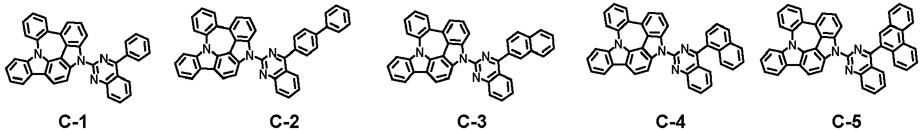

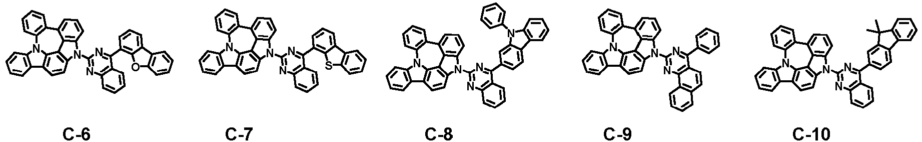

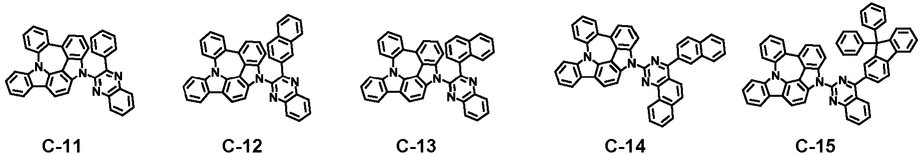

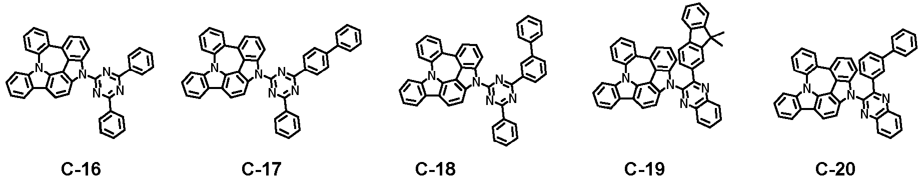

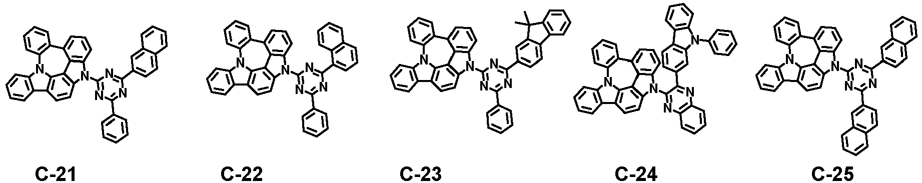

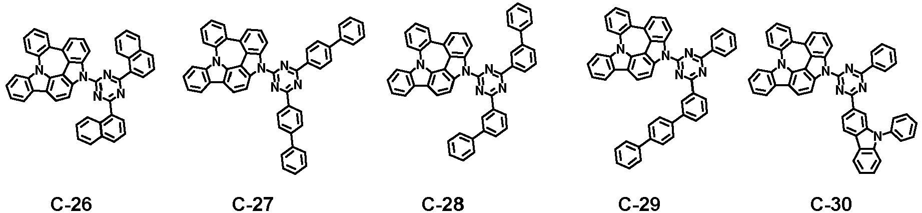

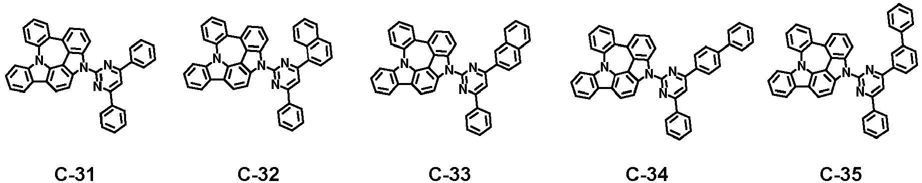

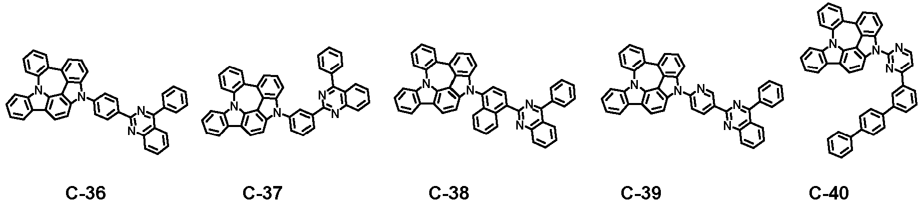

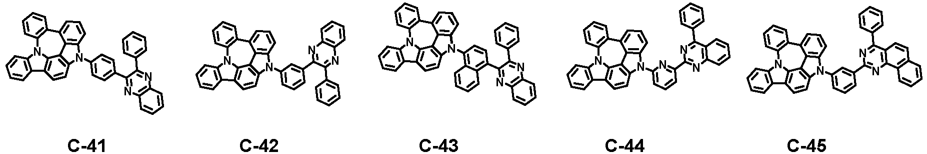

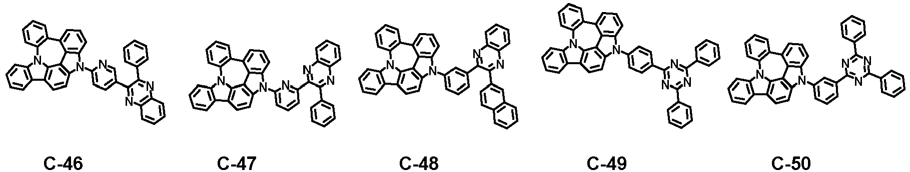

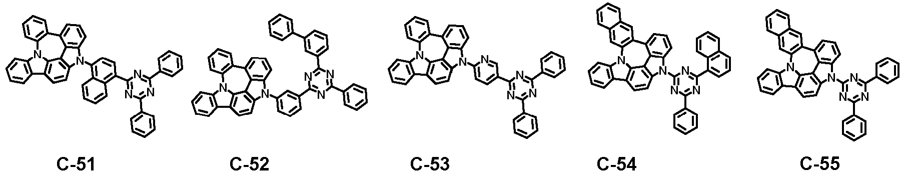

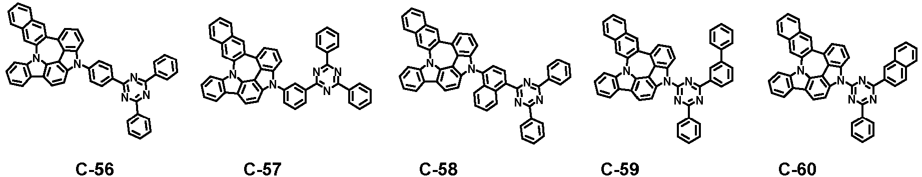

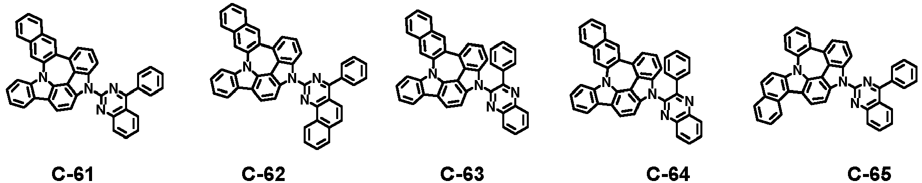

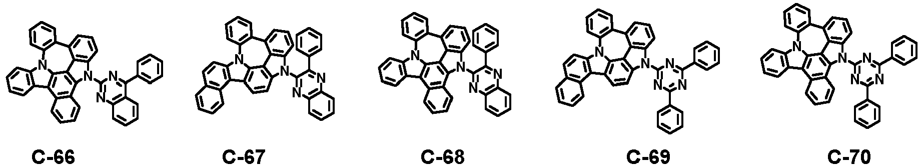

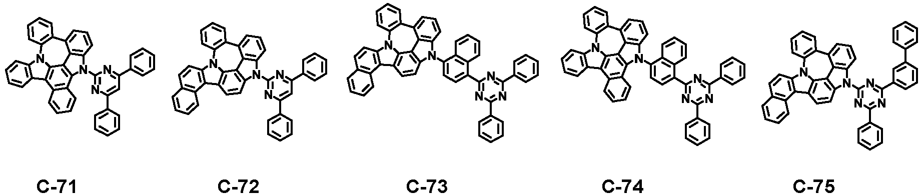

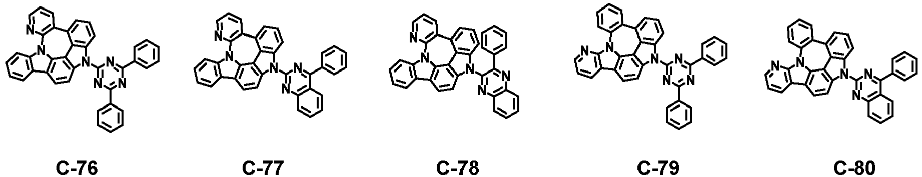

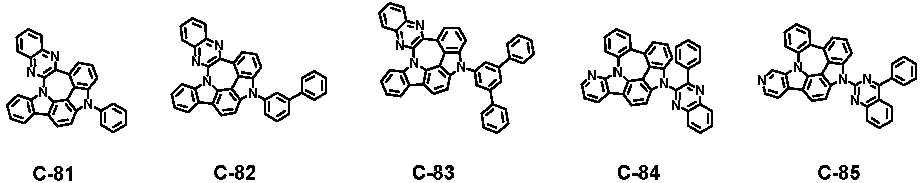

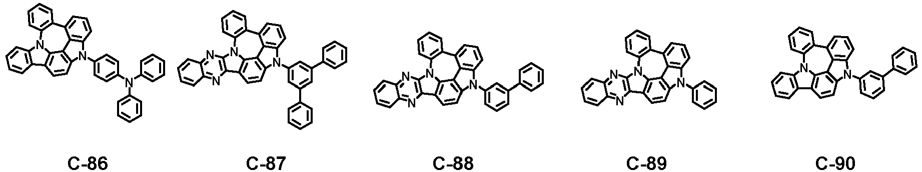

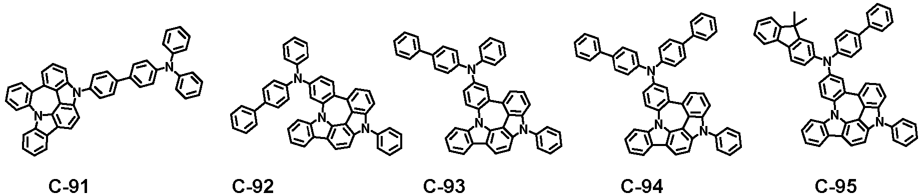

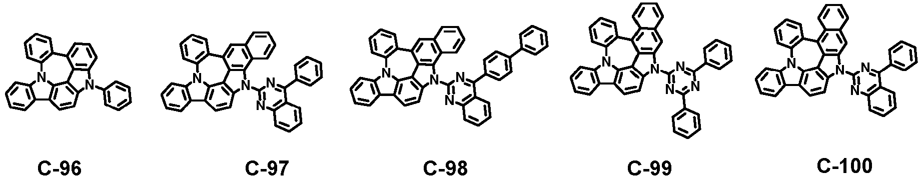

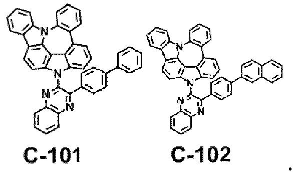

8. An organic electroluminescent material comprising the organic electroluminescent compound according to claim 1.

9. An organic electroluminescent device comprising the organic electroluminescent compound according to claim 1.

10. A display device comprising the organic electroluminescent compound according to claim 1.

## Patentansprüche

1. Organische Elektrolumineszenzverbindung, dargestellt durch die folgende Formel 1: 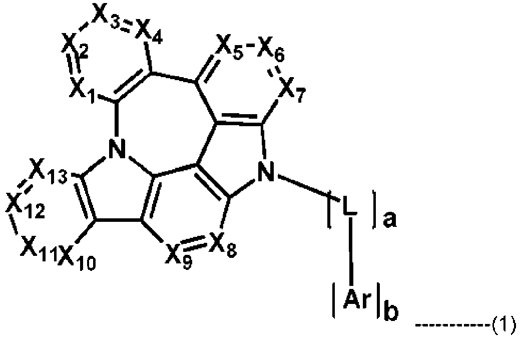 wobei
X₁ bis X₁₀ jeweils CR₁ darstellen;
X₁₁ bis X₁₃ jeweils unabhängig voneinander N oder CR₁ darstellen;
L eine Einfachbindung, ein substituiertes oder unsubstituiertes (C6-C30)-Arylen oder ein substituiertes oder unsubstituiertes 3- bis 30-gliedriges Heteroarylen darstellt;
Ar und R₁ jeweils unabhängig voneinander Wasserstoff, Deuterium, ein Halogen, ein Cyano, ein substituiertes oder unsubstituiertes (C1-C30)-Alkyl, ein substituiertes oder unsubstituiertes (C6-C30)-Aryl, ein substituiertes oder unsubstituiertes 3- bis 30-gliedriges Heteroaryl, ein substituiertes oder unsubstituiertes (C3-C30)-Cycloalkyl, ein substituiertes oder unsubstituiertes (C1-C30)-Alkoxy, ein substituiertes oder unsubstituiertes Tri(C1-C30)-alkylsilyl, ein substituiertes oder unsubstituiertes Di(C1-C30)-alkyl(C6-C30)-arylsilyl, ein substituiertes oder unsubstituiertes (C1-C30)-Alkyldi(C6-C30)-arylsilyl, ein substituiertes oder unsubstituiertes Tri(C6-C30)-arylsilyl, ein substituiertes oder unsubstituiertes Mono- oder Di-(C1-C30)-alkylamino, ein substituiertes oder unsubstituiertes Mono- oder Di-(C6-C30)-arylamino, oder ein substituiertes oder unsubstituiertes (C1-C30)-Alkyl(C6-C30)-arylamino darstellen; oder mit einem benachbarten Substituenten verknüpft sind, um einen substituierten oder unsubstituierten, mono- oder polycyclischen, (C3-C30) alicyclischen oder aromatischen Ring zu bilden, dessen Kohlenstoffatom(e) durch mindestens ein Heteroatom, ausgewählt aus Stickstoff, Sauerstoff und Schwefel, ersetzt sein kann (können);
das Heteroaryl(en) mindestens ein Heteroatom, ausgewählt aus B, N, O, S, Si und P, enthält; und
a und b jeweils unabhängig voneinander eine ganze Zahl von 1 bis 2 darstellen.

2. Organische Elektrolumineszenzverbindung nach Anspruch 1, wobei die Substituenten das substituierte (C1-C30)-Alkyl(en), das substituierte (C6-C30)-Aryl(en), das substituierte 3- bis 30-gliedrige Heteroaryl(en), das substituierte (C3-C30)-Cycloalkyl(en), das substituierte (C1-C30)-Alkoxy, das substituierte Tri(C1-C30)-alkylsilyl, das substituierte Di(C1-C30)-alkyl(C6-C30)-arylsilyl, das substituierte (C1-C30)-Alkyldi(C6-C30)-arylsilyl, das substituierte Tri(C6-C30)-arylsilyl, das substituierte Mono- oder Di-(C1-C30)-alkylamino, das substituierte Mono- oder Di-(C6-C30)-arylamino, das substituierte (C1-C30)-Alkyl(C6-C30)-arylamino und den substituierten mono- oder polycyclischen, (C3-C30) alicyclischen oder aromatischen Ringe in Ar darstellen, L und R1 jeweils unabhängig voneinander mindestens eines sind, ausgewählt aus der Gruppe, bestehend aus Deuterium, einem Halogen, einem Cyano, einem Carboxyl, einem Nitro, einem Hydroxyl, einem (C1-C30)-Alkyl, einem Halogen(C1-C30)-alkyl, einem (C2-C30)-Alkenyl, (C2-C30)-Alkinyl, einem (C1-C30)-Alkoxy, einem (C1-C30)-Alkylthio, einem (C3-C30)-Cycloalkyl, einem (C3-C30)-Cycloalkenyl, einem 3-bis 7-gliedrigem Heterocycloalkyl, einem (C6-C30)-Aryloxy, einem (C6-C30)-Arylthio, einem 5- bis 30-gliedrigem Heteroaryl, unsubstituiert oder substituiert mit einem (C6-C30)-Aryl, einem (C6-C30)-Aryl, unsubstituiert oder substituiert mit einem 5- bis 30-gliedrigen Heteroaryl, einem Tri(C1-C30)-alkylsilyl, einem Tri(C6-C30)-arylsilyl, einem Di(C1-C30)-alkyl(C6-C30)-arylsilyl, einem (C1-C30)-Alkyldi(C6-C30)-aylsilyl, einem Amino, einem Mono- oder Di-(C1-C30)-Alkylamino, ein Mono- oder Di-(C6-C30)-Arylamino, unsubstituiert oder substituiert mit einem (C1-C30)-Alkyl, einem (C1-C30)-Alkyl(C6-C30)-arylamino, einem (C1-C30)-Alkylcarbonyl, einem (C1-C30)-Alkoxycarbonyl, einem (C6-C30)-Arylcarbonyl, einem Di(C6-C30)-Arylboronyl, einem Di(C1- C30)-Alkylboronyl, einem (C1-C30)-Alkyl(C6-C30)-Arylboronyl, einem (C6-C30)-Aryl(C1-C30)-Alkyl und einem (C1-C30)-Alkyl(C6-C30)-Aryl.

3. Organische Elektrolumineszenzverbindung nach Anspruch 1, wobei Formel 1 durch eine der folgenden Formeln 2 bis 7 dargestellt wird: 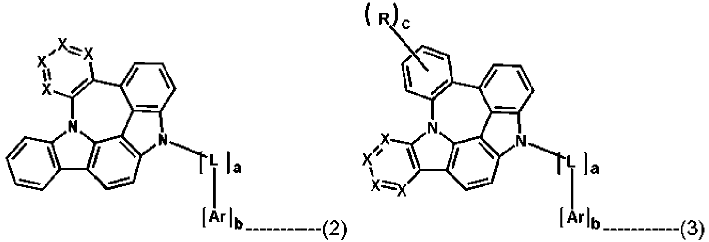 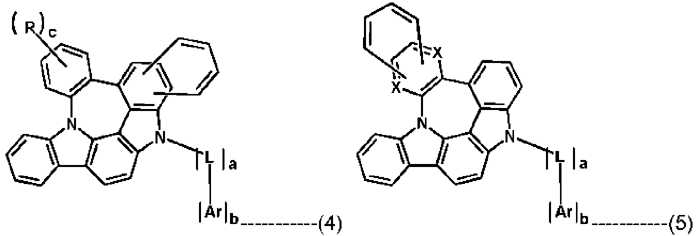 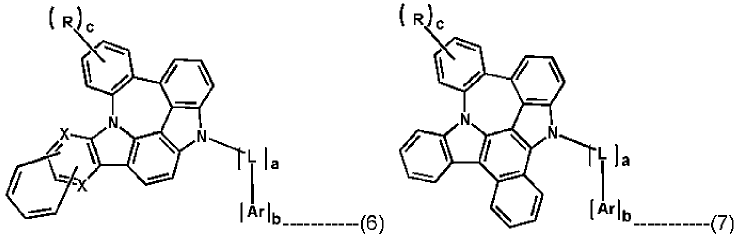 wobei
X CR₁ darstellt;
R ein substituiertes oder unsubstituiertes Mono- oder Di-(C6-C30)-Arylamino darstellt;
c eine ganze Zahl von 1 bis 2 darstellt;
L, Ar, R₁, a und b wie in Anspruch 1 definiert sind.

4. Organische Elektrolumineszenzverbindung nach Anspruch 1, wobei
X₁ bis X₁₀ jeweils CR₁ darstellen;
X₁₁ bis X₁₃ jeweils unabhängig voneinander N oder CR₁ darstellen;
L eine Einfachbindung, ein substituiertes oder unsubstituiertes (C6-C15)-Arylen oder ein substituiertes oder unsubstituiertes 5- bis 15-gliedriges Heteroarylen darstellt;
Ar Wasserstoff, ein substituiertes oder unsubstituiertes (C6-C20)-Aryl, ein substituiertes oder unsubstituiertes 5- bis 20-gliedriges Heteroaryl oder ein substituiertes oder unsubstituiertes Di(C6-C15)-Arylamino darstellt; und
R₁ Wasserstoff oder ein substituiertes oder unsubstituiertes Di(C6-C15)-arylamino darstellt; oder mit einem benachbarten Substituenten verbunden ist, um einen substituierten oder unsubstituierten, mono- oder polycyclischen, (C3-C15) alicyclischen oder aromatischen Ring zu bilden, dessen Kohlenstoffatom(e) durch mindestens ein Heteroatom, ausgewählt aus Stickstoff, Sauerstoff und Schwefel, ersetzt sein kann (können).

5. Organische Elektrolumineszenzverbindung nach Anspruch 1, wobei
X₁ bis X₁₀ jeweils CR₁ darstellen;
X₁₁ bis X₁ jeweils unabhängig voneinander N oder CR₁ darstellen;
L eine Einfachbindung, ein unsubstituiertes (C6-C15)-Arylen oder ein unsubstituiertes 5- bis 15-gliedriges Heteroarylen darstellt;
Ar Wasserstoff; ein unsubstituiertes (C6-C20)-Aryl; ein 5- bis 20-gliedriges Heteroaryl, unsubstituiert oder substituiert mit einem (C1-C6)-Alkyl, einem (C6-C25)-Aryl, einem 5- bis 20-gliedrigen Heteroaryl oder einem (C1-C6)-Alkyl(C6-C15)aryl; oder ein unsubstituiertes Di(C6-C15)-Arylamino darstellt; und
R₁ Wasserstoff oder ein Di(C6-C15)-Aylamino, unsubstituiert oder substituiert mit einem (C1-C6)-Alkyl, darstellt; oder mit einem benachbarten Substituenten verbunden ist, um einen unsubstituierten monocyclischen (C3-C15) aromatischen Ring zu bilden.

6. Organische Elektrolumineszenzverbindung nach Anspruch 1, wobei Ar ein substituiertes oder unsubstituiertes Phenyl, ein substituiertes oder unsubstituiertes Naphthyl, ein substituiertes oder unsubstituiertes Biphenyl, ein substituiertes oder unsubstituiertes Terphenyl, ein substituiertes oder unsubstituiertes Triazinyl, ein substituiertes oder unsubstituiertes Pyrimidinyl, ein substituiertes oder unsubstituiertes Chinazolinyl, ein substituiertes oder unsubstituiertes Benzochinazolinyl, ein substituiertes oder unsubstituiertes Chinoxalinyl, ein substituiertes oder unsubstituiertes Benzochinoxalinyl, ein substituiertes oder unsubstituiertes Chinolyl, ein substituiertes oder unsubstituiertes Benzochinolyl, ein substituiertes oder unsubstituiertes Isochinolyl, ein substituiertes oder unsubstituiertes Benzoisochinolyl, ein substituiertes oder unsubstituiertes Triazolyl, ein substituiertes oder unsubstituiertes Pyrazolyl, ein substituiertes oder unsubstituiertes Dibenzothiophenyl, ein substituiertes oder unsubstituiertes Benzothiophenyl, ein substituiertes oder unsubstituiertes Dibenzofuranyl oder ein substituiertes oder unsubstituiertes Benzofuranyl ist.

7. Organische Elektrolumineszenzverbindung nach Anspruch 1, wobei die Verbindung durch die Formel 1 dargestellt ist, ausgewählt ist aus der Gruppe bestehend aus:

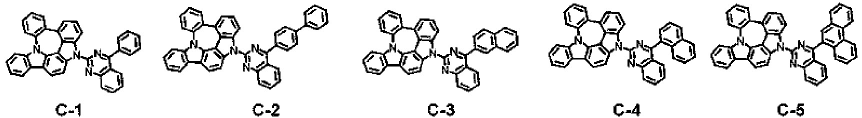

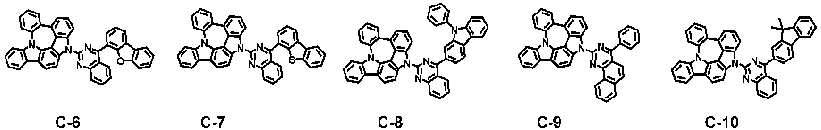

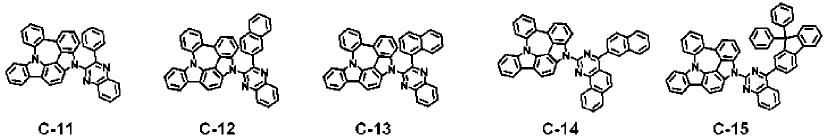

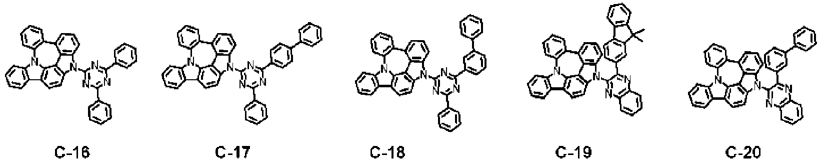

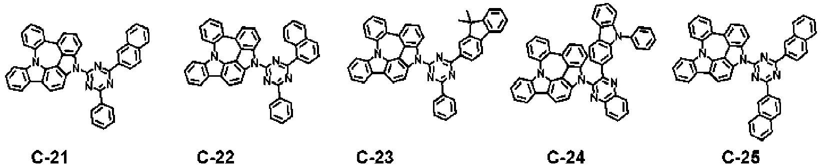

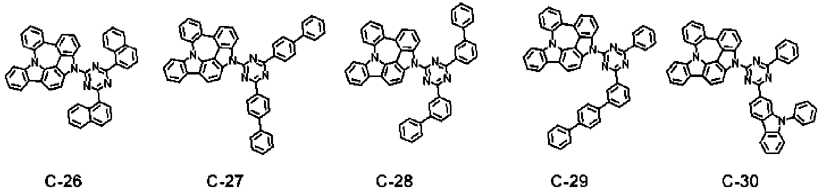

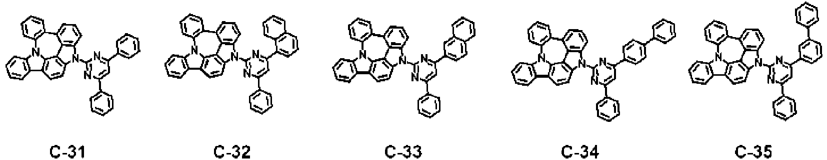

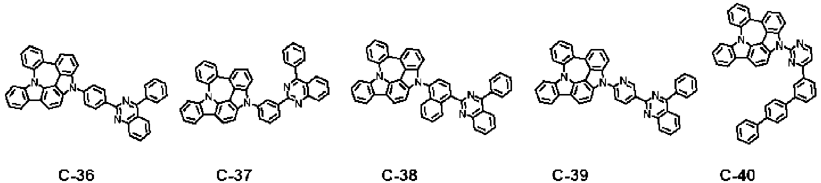

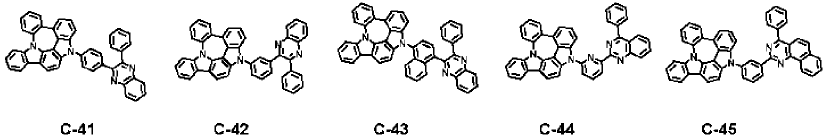

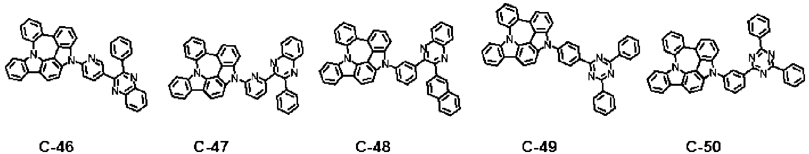

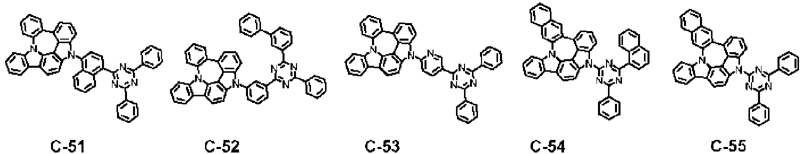

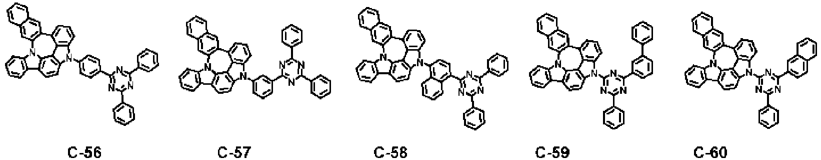

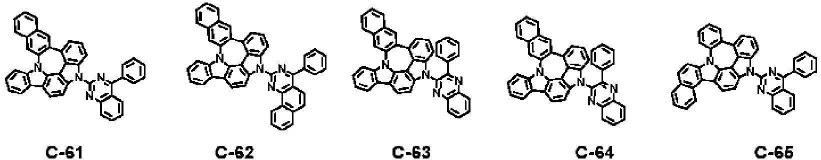

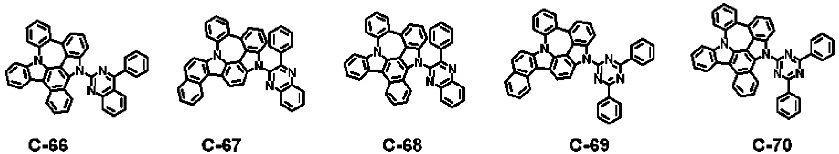

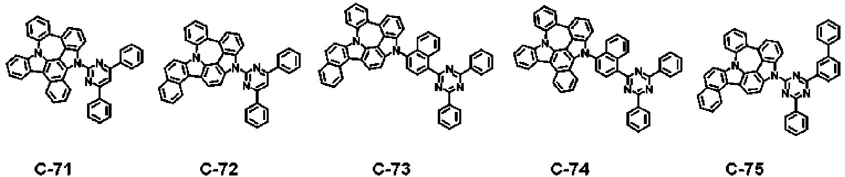

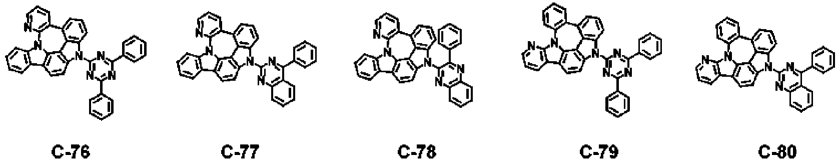

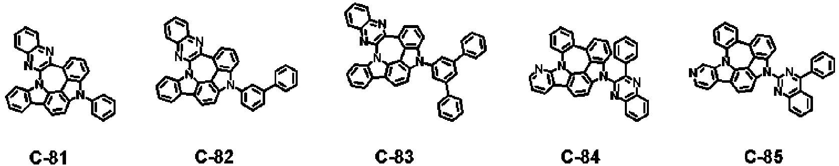

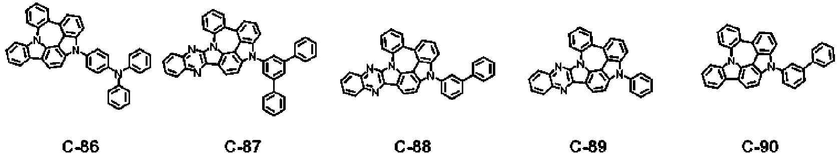

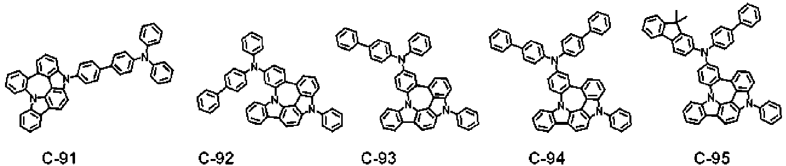

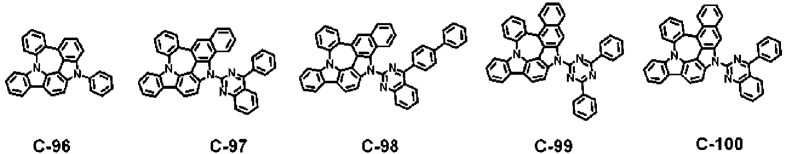

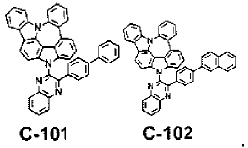

8. Organisches Elektrolumineszenzmaterial, umfassend die organische Elektrolumineszenzverbindung nach Anspruch 1.

9. Organische Elektrolumineszenzvorrichtung, umfassend die organische Elektrolumineszenzverbindung nach Anspruch 1.

10. Anzeigevorrichtung, umfassend die organische Elektrolumineszenzverbindung nach Anspruch 1.

## Revendications

1. Composé électroluminescent organique représenté par la formule 1 suivante : 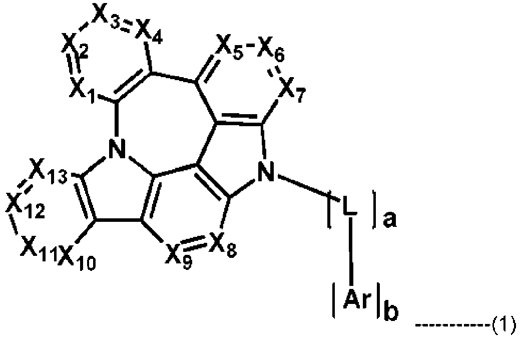 dans laquelle
X₁ à X₁₀ représentent chacun CR₁ ;
X₁₁ à X₁₃ représentent chacun indépendamment N ou CR₁ ;
L représente une liaison simple, un (C6-C30)arylène substitué ou non substitué, ou un hétéroarylène à 3 à 30 chaînons substitué ou non substitué ;
Ar et R₁ représentent chacun indépendamment l'hydrogène, le deutérium, un halogène, un cyano, un (C1-C30)alkyle substitué ou non substitué, un (C6-C30)aryle substitué ou non substitué, un hétéroaryle à 3 à 30 chaînons substitué ou non substitué, un (C3-C30)cycloalkyle substitué ou non substitué, un (C1-C30)alkoxy substitué ou non substitué, un tri(C1-C30)alkylsilyle substitué ou non substitué, un di(C1-C30)alkyl(C6- C30)arylsilyle substitué ou non substitué, un (C1-C30)alkyldi(C6-C30)arylsilyle substitué ou non substitué, un tri(C6-C30)arylsilyle substitué ou non substitué, un mono- ou di-(C1-C30)alkylamino substitué ou non substitué, un mono- ou di-(C6-C30)arylamino substitué ou non substitué ou un (C1-C30)alkyl(C6-C30)arylamino substitué ou non substitué ; ou sont liés à un substituant adjacent pour former un cycle alicyclique ou aromatique (C3-C30) mono-ou polycyclique substitué ou non substitué, dont l'atome/les atomes de carbone peu(ven)t être remplacé(s) par au moins un hétéroatome choisi parmi l'azote, l'oxygène et le soufre ;
l'hétéroaryl(ène) contient au moins un hétéroatome choisi parmi B, N, O, S, Si et P ; et
a et b représentent chacun indépendamment un entier de 1 à 2.

2. Composé électroluminescent organique selon la revendication 1, dans lequel les substituants du (C1-C30)alkyl(ène) substitué, (C6-C30)aryl(ène) substitué, hétéroaryl(ène) à 3-30-chaînons substitué, (C3-C30)cycloalkyl(ène) substitué, (C1-C30)alcoxy substitué, tri(C1-C30)alkylsilyle substitué, di(C1- C30)alkyl(C6-C30)arylsilyle substitué, (C1-C30)alkyldi(C6-C30)arylsilyle substitué, tri(C6-C30)arylsilyle substitué, mono- ou di-(C1-C30)alkylamino substitué, mono- ou di- (C6-C30)arylamino substitué, (C1-C30)alkyl(C6-C30)arylamino substitué et du cycle alicyclique ou aromatique (C3-C30) mono- ou polycyclique dans Ar, L et R1 sont chacun indépendamment au moins un choisi dans le groupe consistant en le deutérium, un halogène, un cyano, un carboxyle, un nitro, un hydroxyle, un (C1-C30)alkyle, un halo(C1-C30)alkyle, un (C2- C30)alcényle, un (C2-C30)alcynyle, un (C1-C30)alcoxy, un (C1-C30)alkylthio, un (C3- C30)cycloalkyle, un (C3-C30)cycloalcényle, un hétérocycloalkyle à 3 à 7 chaînons, un (C6-C30)aryloxy, un (C6-C30)arylthio, un hétéroaryle à 5 à 30 chaînons non substitué ou substitué par un (C6-C30)aryle, un (C6-C30)aryle non substitué ou substitué par un hétéroaryle à 5 à 30 chaînons, un tri(C1-C30)alkylsilyle, un tri(C6-C30)arylsilyle, un di(C1-C30)alkyl(C6- C30)arylsilyle, un (C1-C30)alkyldi(C6-C30)arylsilyle, un amino, un mono- ou di- (C1- C30)alkylamino, un mono- ou di- (C6-C30)arylamino non substitué ou substitué par un (C1-C30)alkyle, un (C1-C30)alkyl(C6-C30)arylamino, un (C1-C30)alkylcarbonyle, un (C1-C30)alkoxycarbonyle, un (C6-C30)arylcarbonyle, un di(C6-C30)arylboronyle, un di(C1-C30)alkylboronyle, un (C1-C30)alkyl(C6-C30)arylboronyle, un (C6-C30)aryl(C1-C30)alkyle et un (C1 - C30)alkyl(C6-C30)aryle.

3. Composé électroluminescent organique selon la revendication 1, dans lequel la formule 1 est représentée par l'une quelconque des formules 2 à 7 suivantes : 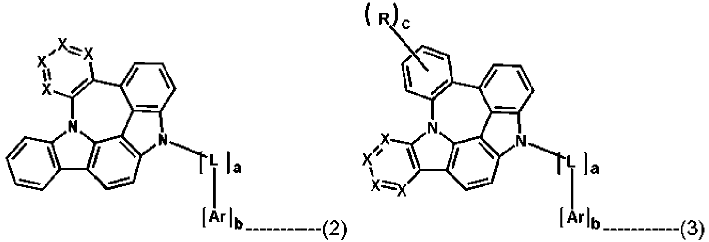 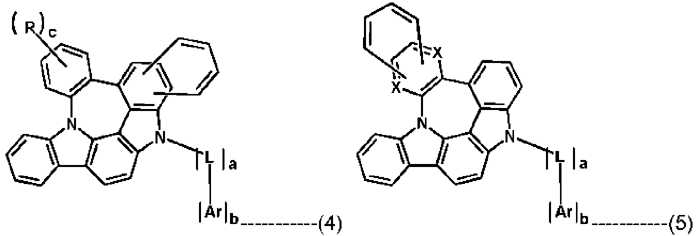 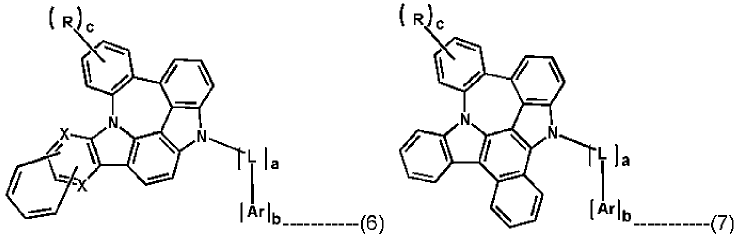 dans lesquelles
X représente CR₁ ;
R représente un mono- ou di- (C6-C30)arylamino substitué ou non substitué ;
c représente un entier de 1 à 2 ; et
L, Ar, R₁, a et b sont tels que définis dans la revendication 1.

4. Composé électroluminescent organique selon la revendication 1, dans lequel
X₁ à X₁₀ représentent chacun CR₁ ;
X₁₁ à X₁₃représentent chacun indépendamment N ou CR₁ ;
L représente une liaison simple, un (C6-C15)arylène substitué ou non substitué, ou un hétéroarylène à 5 ou 15 chaînons substitué ou non substitué ;
Ar représente l'hydrogène, un (C6-C20)aryle substitué ou non substitué, un hétéroaryle à 5 à 20 chaînons substitué ou non substitué ou un di(C6-C15)arylamino substitué ou non substitué ; et
R₁ représente l'hydrogène, ou un di(C6-C15)arylamino substitué ou non substitué ; ou est lié à un substituant adjacent pour former un cycle alicyclique ou aromatique (C3-C15) mono- ou polycyclique substitué ou non substitué, dont l'atome/les atomes de carbone peu(ven)t être remplacé(s) par au moins un hétéroatome choisi parmi l'azote, l'oxygène et le soufre.

5. Composé électroluminescent organique selon la revendication 1, dans lequel
X₁ à X₁₀ représentent chacun CR₁ ;
X₁₁ à X₁₃ représentent chacun indépendamment N ou CR₁ ;
L représente une liaison simple, un (C6-C15)arylène non substitué ou un hétéroarylène non substitué à 5 à 15 chaînons ;
Ar représente l'hydrogène ; un (C6-C20)aryle non substitué ; un hétéroaryle à 5 à 20 chaînons non substitué ou substitué par un (C1-C6) alkyle, un (C6-C25)aryle, un hétéroaryle à 5 à 20 chaînons ou un alkyl(C1-C6)(C6-C15)aryle ou un di(C6-C15)arylamino non substitué ; et
R₁ représente l'hydrogène, ou un di(C6-C15)arylamino non substitué ou substitué par un (C1-C6)alkyle ; ou est lié à un substituant adjacent pour former un cycle aromatique (C3-C15) monocyclique non substitué.

6. Composé électroluminescent organique selon la revendication 1, dans lequel Ar représente un phényle substitué ou non substitué, un naphtyle substitué ou non substitué, un biphényle substitué ou non substitué, un terphényle substitué ou non substitué, un triazinyle substitué ou non substitué, un pyrimidinyle substitué ou non substitué, un quinazolinyle substitué ou non substitué, un benzoquinazolinyle substitué ou non substitué, un quinoxalinyle substitué ou non substitué, un benzoquinoxalinyle substitué ou non substitué, un quinolyle substitué ou non substitué, un benzoquinolyle substitué ou non substitué, un isoquinolyle substitué ou non substitué, un benzoisoquinolyle substitué ou non substitué, un triazolyle substitué ou non substitué, un pyrazolyle substitué ou non substitué, un dibenzothiophényle substitué ou non substitué, un benzothiophényle substitué ou non substitué, un dibenzofuranyle substitué ou non substitué ou un benzofuranyle substitué ou non substitué.

7. Composé électroluminescent organique selon la revendication 1, dans lequel le composé représenté par la formule 1 est choisi dans le groupe consistant en :

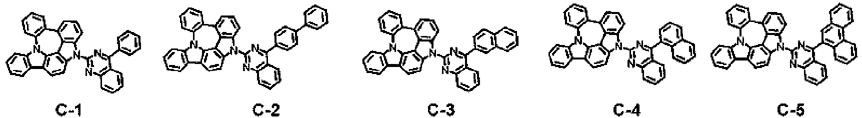

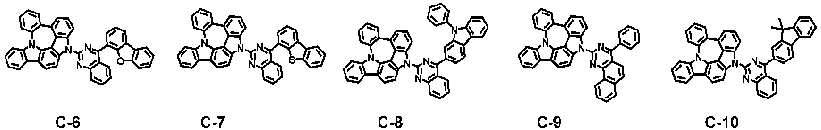

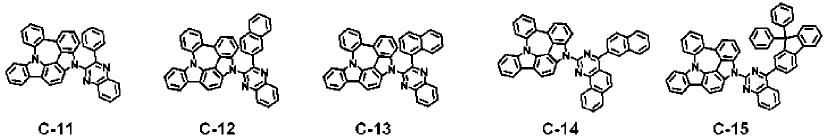

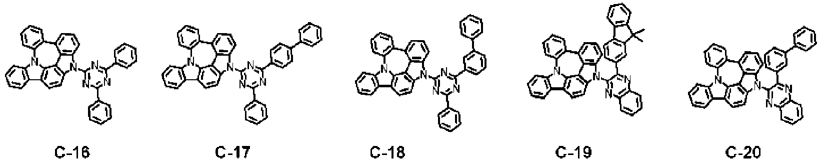

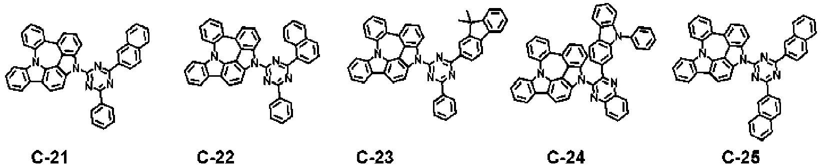

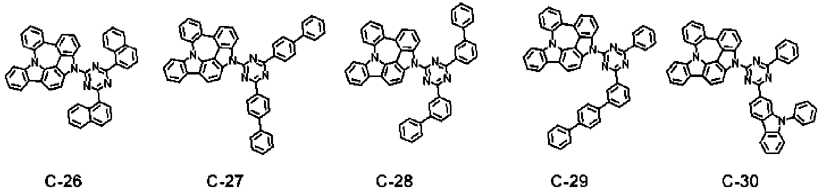

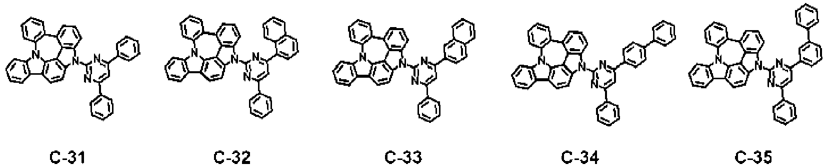

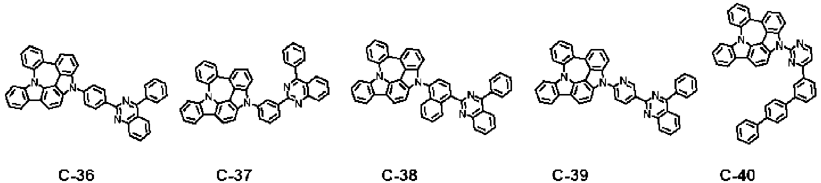

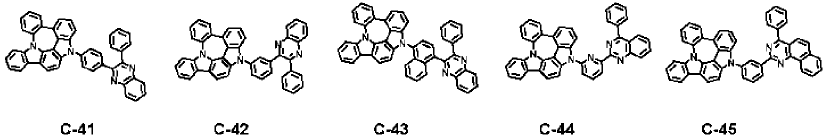

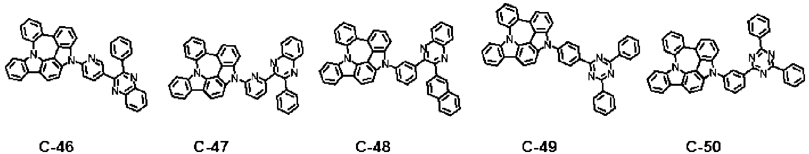

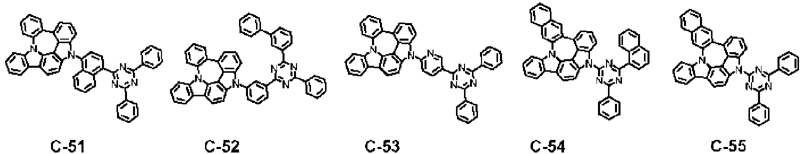

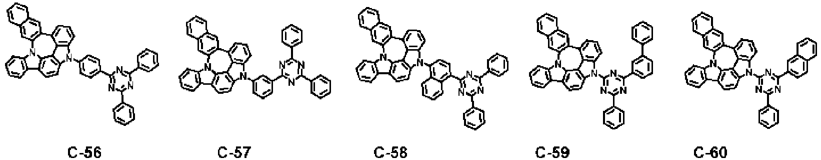

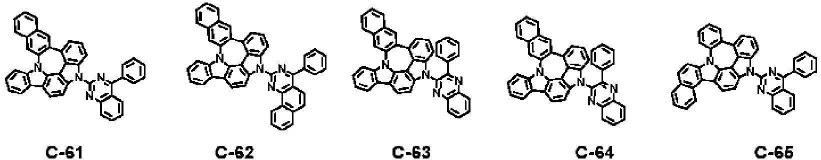

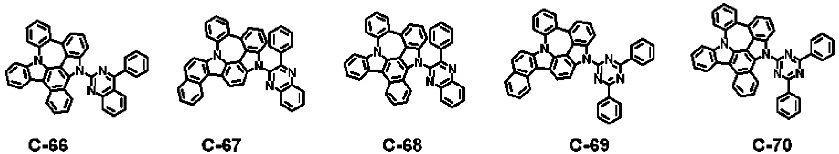

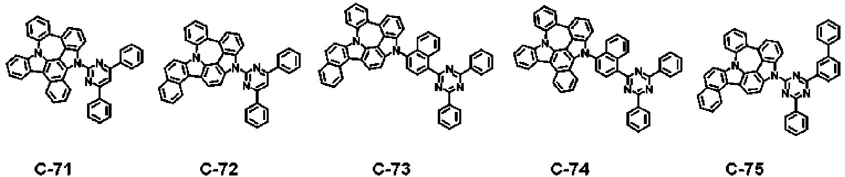

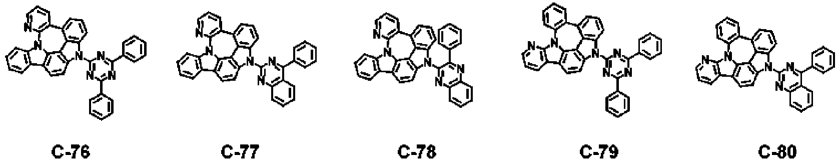

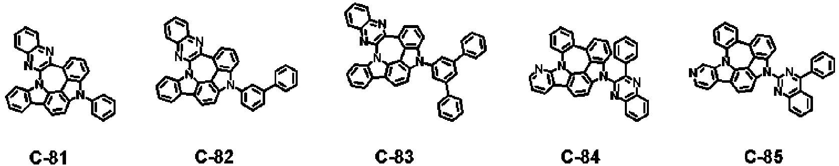

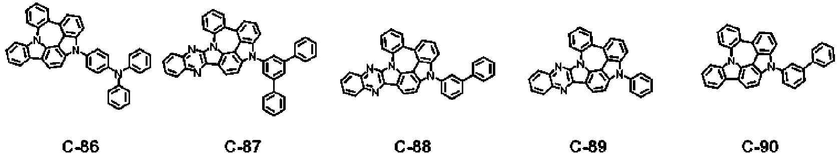

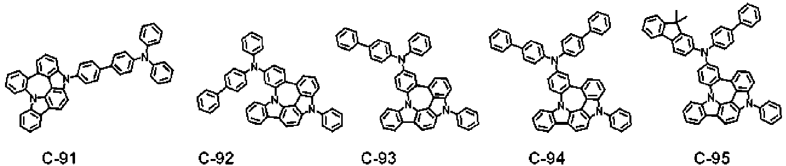

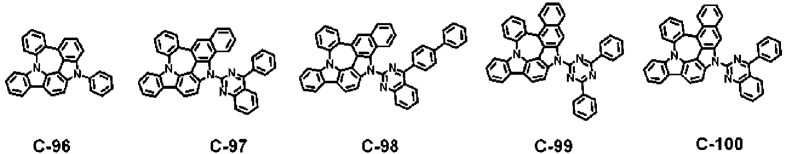

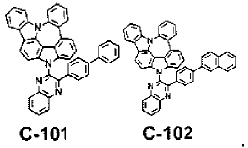

8. Matériau électroluminescent organique comprenant le composé électroluminescent organique selon la revendication 1.

9. Dispositif électroluminescent organique comprenant le composé électroluminescent organique selon la revendication 1.

10. Dispositif d'affichage comprenant le composé électroluminescent organique selon la revendication 1.
